# EUROPÄISCHE PATENTSCHRIFT

(11) **EP 1 738 737 B1**
(45) Veröffentlichungstag und Bekanntmachung des Hinweises auf die Patenterteilung: **16.05.2012**
(21) Anmeldenummer: 06004915.2
(22) Anmeldetag: 10.03.2006
(51) Int. Cl.: A61K 6/10, A61K 6/093

(54) **Zu formstabilen Formkörpern aushärtendes kondensationsvernetzendes Dentalmaterial**
Dimensionally stable articles from cured condensation-crosslinked dental material
Articles indéformables de matériau dentaire réticulant par condensation

(30) Priorität: 01.07.2005 DE 102005031201
(43) Veröffentlichungstag der Anmeldung: 03.01.2007
(73) Patentinhaber: Kettenbach GmbH & CO. KG, 35713 Eschenburg (DE)
(72) Erfinder: Bublewitz, Alexander. Dr, 35745 Herborn (DE); Reber, Jens-Peter. Dr, 58540 Meinerzhagen (DE)
(74) Vertreter: KEIL & SCHAAFHAUSEN Patentanwälte

(56) Entgegenhaltungen:
- EP-A- 1 402 873
- EP-A2- 0 748 621
- EP-A2- 1 226 808
- WO-A-2005/077321

## Beschreibung

Die vorliegende Erfindung betrifft kondensationsvernetzende Zweikomponenten-Dentalabform-materialien, auf Basis von alkoxysilylfunktionellen und/oder hydroxysilylfunktionellen Polyethern, welche insbesondere zur Abdrucknahme geeignet sind, und deren Verwendung. Derartige Materialien werden in der Dentalmedizin bspw. zur Zahnabdrucknahme, Bissregistrierung, Zahnprothesenunterfütterung, als provisorischer und permanenter Dentalzement, provisorisches Verschlussmaterial oder dentales Zahntechnik-Dubliermaterial eingesetzt.

Bekannte kondensationsvernetzende Dentalmaterialien enthalten üblicherweise hydroxylfunktionelle Polymere mit einem Siliconrückgrat, welche in Gegenwart organischer Zinnverbindungen als Katalysatoren, Alkoxysilanen und/oder Kieselsäurestern als Vernetzer und Wasser aushärten. Allerdings sind derartige Materialien aufgrund des Siliconrückgrats der Polymere vergleichsweise hydrophob, so dass diesen zwecks Herabsetzung der Oberflächenspannung und zur Einstellung der erforderlichen Benetzbarkeit erhebliche Anteile an Tensiden zugefügt werden müssen. Ein weiterer Nachteil dieser Zusammensetzungen liegt in dem Einsatz toxikologisch bedenklicher organischer Zinnverbindungen als Katalysator.

Alternativ dazu sind Zweikomponenten-Dentalmaterialien bekannt, welche terminale Alkoxysilylgruppen aufweisende Polymere mit einem hydrophilen Polyetherrückgrat enthalten, die zur Benetzung der feuchten Zahnsubstanz ausreichend hydrophile Eigenschaften aufweisen. Üblicherweise bestehen diese Materialien aus einer Basiskomponente enthaltend alkoxysilylfunktionelle und/oder hydroxysilylfunktionelle Polyether mit einem mittleren Molekulargewicht von 800 bis 20.000 g/mol, welche synthesebedingt auch Harnstoff- und/oder Urethangruppen aufweisen können, Füllstoffe sowie ggf. weitere Zusatzstoffe, und einer Katalysatorkomponente, welche neben Füll- und ggf. weiteren Hilfsstoffen eine organische und/oder anorganische Säure als Katalysator enthält. Derartige Materialien, welche bspw. aus der EP 0 269 819 B1 und EP 1 226 808 A2 bekannt sind, weisen jedoch aufgrund der Säurekatalyse unbefriedigende anwendungstechnische Eigenschaften auf.

Aus diesem Grund wurden bereits basen- oder salzkatalysiert aushärtende Materialien auf Basis von alkoxysilylfunktionellen Polyethern vorgeschlagen. In der WO 99/48942 werden als Katalysator zur Vernetzung von als Kleb- oder Dichtstoffen einzusetzenden und Polyethergruppen aufweisenden Polyurethanen metalllorganische Verbindungen, wie Eisen- oder Zinnverbindungen, bspw. Zinn(II)oktat, oder tertiäre Amine, wie Triethylamin, einzusetzen. Die ausgehärteten Vulkanisate/Formkörper dieser Dentalmaterialien neigen jedoch dazu, nach einer gewissen Lagerzeit bei Raumtemperatur und insbesondere bei Lagerung bei erhöhter Temperatur (z.B. 60°C) durch Umkehr der Vernetzungsreaktion (Rückspaltung) ihre ursprüngliche Form zu verlieren und im Extremfall unter Zersetzung zu zerfließen. Sie werden dadurch als Dentalabformmaterial unbrauchbar, denn der fertige Abdruck kann häufig erst nach einiger Zeit nach Abdrucknahme zur Herstellung eines Zahnmodells weiterverarbeitet werden oder der Abdruck muss nach einer längeren Zeit (zum Teil mehrmals) erneut zur Erstellung eines Zahnmodells verwendet werden. Verformte oder zerflossene Abdrücke verfälschen hierbei das Zahnmodell und führen zu nicht brauchbarem Zahnersatz.

Aufgabe der vorliegenden Erfindung ist es daher, ein basen- oder salzkatalysiertes hydrophiles, kondensationsvernetzendes Dentalabformmaterial auf Basis von Alkoxysilylpolyethern und/oder Hydroxysilylpolyethern zur Verfügung zu stellen, dass nach dessen Aushärtung auch nach längerer Lagerung, insbesondere einer längeren Lagerung bei erhöhten Temperaturen, keine Rückspaltung aufweist, formstabil ist und alle Anforderungen an ein Dentalmaterial erfüllt.

Erfindungsgemäß wird diese Aufgabe durch ein kondensationsvernetzendes Zweikomponenten-Dentalmaterial mit der Zusammensetzung gemäß Patentanspruch 1 gelöst.

Überraschenderweise konnte im Rahmen der vorliegenden Erfindung herausgefunden werden, dass durch den Zusatz einer sauren Verbindung mit einer bei 20°C gemessenen Wasserlöslichkeit von nicht mehr als 150 g/l in ein basen- oder salzkatalysiert aushärtendes Dentalmaterial auf Basis von alkoxysilylfunktionellen und/oder hydroxysilylfunktionellen Polyethern eine Umkehr der Vernetzungsreaktion bzw. Rückspaltung, wie sie bei den aus dem Stand der Technik bekannten basen- und salzkatalysierten Materialien zwangsläufig auftritt, weil die durch die freie Basen oder durch die Salze katalysierte Kondensationsreaktion reversibel ist und die Katalysatoren nach der Vernetzungsreaktion im ausgehärteten Zustand weiterhin vorhanden und aktiv sind und deshalb eine Rückreaktion unter Spaltung der vormals gebildeten Si-O-Si-Bindungen katalysieren, zuverlässig verhindert wird. Daher weisen die erfindungsgemäßen Dentalmaterialien nach Aushärtung selbst nach einer Lagerung von mehreren Monaten - auch bei erhöhten Temperaturen - unter Beibehaltung der grundlegenden Anforderungen an ein dentales Abformmaterial, wie z.B. Lagerstabilität der Einzelkomponenten über einen Zeitraum von mindestens 12 Monaten, ausreichende Verarbeitungszeit, möglichst kurze Abbindezeit, toxikologische Unbedenklichkeit und alle anderen Anforderungen wie sie aus der ISO 4823 (August 2001) hervorgehen, eine hohe Formstabilität auf. Zudem hat sich gezeigt, dass trotz des Zusatzes der erfindungsgemäßen sauren Verbindung in einem basen- oder salzkatalysiert aushärtenden Dentalmaterial auf Basis von alkoxysilylfunktionellen und/oder hydroxysilylfunktionellen Polyethern keine spontane Beschleunigung bzw. Verlangsamung der Aushärtereaktion auftritt. Dies war insbesondere deshalb unerwartet, weil Säuren bekanntermaßen die Aushärtung von Alkoxysilyl- bzw. Hydroxysilylpolyethern katalysieren, was eine Beschleunigung der Aushärtereaktion in säurekatalysierten System erwarten lassen würde, und Basen bzw. Salze als Katalysator enthaltende Alkoxysilylpolyethermischungen mit sauren Verbindungen eine Neutralisationsreaktion eingehen können, was zwangsläufig zu einer Verlangsamung der Aushärtung führt. Nach den Erkenntnissen der vorliegenden Erfindung wird eine vorzeitige säurekatalysierte Aushärtung bzw. eine Neutralisationsreaktion durch die geringe Löslichkeit der erfindungsgemäß eingesetzten sauren Verbindung verhindert.

Erfindungsgemäß sind die Dentalmaterialien als Zweikomponentenmaterial formuliert. Dabei ist der Katalysatorkomponente des erfindungsgemäßen Zweikomponenten-Dentalmaterials vorzugsweise Wasser zugesetzt.

Die erfindungsgemäßen Zweikomponenten-Dentalmaterialien werden so formuliert, dass die

### Basiskomponente A

a) wenigstens einen alkoxysilylfunktionellen und/oder hydroxysilylfunktionellen Polyether,
   und die Katalysatorkomponente B
b) wenigstens einen aus der aus Basen, Salzen und beliebigen Kombinationen hiervon bestehenden Gruppe ausgewählten Katalysator und
c) Wasser
enthält, wobei die Komponente A zusätzlich mindestens eine saure Verbindung mit einer Wasserlöslichkeit (20°C) von nicht mehr als 150 g/l enthält.

Grundsätzlich kann das erfindungsgemäße Dentalmaterial zusätzlich zu dem aus der aus Basen, Salzen und beliebigen Kombinationen hiervon bestehenden Gruppe ausgewählten Katalysator jede Art von saurer Verbindung d) enthalten, sofern die bei 20°C gemessenen Wasserlöslichkeit der sauren Verbindung d) bzw. aller zugesetzten sauren Verbindungen d) nicht mehr als 150 g/l beträgt. Besonders gute Ergebnisse werden insbesondere erhalten, wenn die wenigstens eine saure Verbindung d) und besonders bevorzugt alle zugesetzten sauren Verbindungen d) aus der aus organischen Säuren, anorganischen Säuren, anorganischen Säureanhydriden, organischen Säureanhydriden und beliebigen Kombinationen hiervon bestehenden Gruppe ausgewählt ist/sind.

In Weiterbildung des Erfindungsgedankens wird vorgeschlagen, wenigstens eine saure Verbindung d) mit einer Wasserlöslichkeit (20°C) von nicht mehr als 50 g/l, bevorzugt von nicht mehr als 1 g/l und besonders bevorzugt von nicht mehr als 0,5 g/l, einzusetzen. Sofern das Dentalmaterial mehrere saure Verbindungen d) aufweist, erfüllen vorzugsweise alle die vorgenannten Kriterien.

Gemäß einer weiteren bevorzugten Ausführungsform der vorliegenden Erfindung weist die wenigstens eine saure Verbindung d) eine Polyetherlöslichkeit (20°C) von nicht mehr als 2 g/l, besonders bevorzugt von nicht mehr als 1 g/l und ganz besonders bevorzugt von nicht mehr als 0,5 g/l, auf. Polyetherlöslichkeit im Sinne der vorliegenden Erfindung bezeichnet die bei 20°C gemessene Löslichkeit einer Verbindung in einem Polyether der nachfolgenden Formel: 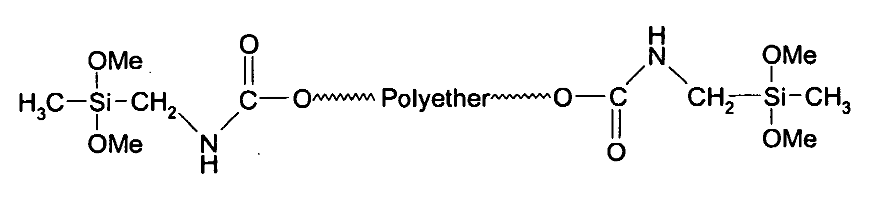 Gute Ergebnisse werden insbesondere erhalten, wenn dem Dentalmaterial wenigstens eine saure Verbindung d) mit einem in Wasser (20°C) gemessenen pKₛ-Wert zwischen 1,0 und 6,5 zugesetzt wird, wobei saure Verbindungen d) mit einem pKₛ-Wert zwischen 1,3 und 5,0 und insbesondere solche mit einem pKₛ-Wert zwischen 1,5 und 4,3 besonders bevorzugt sind. Sofern mehrwertige Säuren eingesetzt werden, reicht es aus, wenn wenigstens einer der pKₛ-Werte, vorzugsweise der pKₛ₁-Wert, der eingesetzten mehrwertigen Säure in den vorgenannten Bereichen liegt. Bei Einsatz von Säureanhydriden ist der pKₛ-Wert der dem Anhydrid zugrundeliegenden Säure maßgeblich, im Falle von Bernsteinsäureanhydrid bspw. der pKₛ-Wert von Bernsteinsäure.

Beispiele für geeignete saure Verbindungen d), welche die vorgenannten Kriterien erfüllen und daher bevorzugt eingesetzt werden, sind Barbitursäure, Bernsteinsäureanhydrid und insbesondere Ethylendiamintetraessigsäure (CAS-Nr. 60-00-4), Derivate von Ethylendiamintetraessigsäure, Mono-, Di- und Tri-Alkalisalze von Ethylendiamintetraessigsäure sowie Mono-, Di- und Tri-Erdalkalisalze von Ethylendiamintetraessigsäure. Vorzugsweise enthält das Dentalmaterial nur eine saure Verbindung d), und zwar eine aus der aus Ethylendiamintetraessigsäure, Mono-, Di- und Tri-Alkalisalze von Ethylendiamintetraessigsäure sowie Mono-, Di- und Tri-Erdalkalisalze von Ethylendiamintetraessigsäure bestehenden Gruppe ausgewählte.

Gemäß einer weiteren besonderen Ausführungsform der vorliegenden Erfindung enthält das Zweikomponenten-Dentalmaterial, bezogen auf die Gesamtmischung, 0,001 bis 10 Gew.-%, besonders bevorzugt 0,01 bis 5 Gew.-%, ganz besonders bevorzugt 0,1 bis 2 Gew.-% und höchst bevorzugt 0,2 bis 1,5 Gew.-% wenigstens einer sauren Verbindung d). Besonders gute Ergebnisse werden insbesondere erhalten, wenn das Dentalmaterial nur eine saure Verbindung d) in den vorgenannten Mengenbereichen enthält.

In Weiterbildung des Erfindungsgedankens wird vorgeschlagen, dass Zweikomponenten-Dentalmaterial so zu formulieren, dass dieses nach Aushärtung und anschließender Lagerung bei Raumtemperatur für mindestens 1 Monat, bevorzugt mindestens 3 Monate sowie besonders bevorzugt mindestens 6 Monate und/oder nach Lagerung zwischen 40°C und 60°C für mindestens 1 Woche, bevorzugt mindestens 2 Wochen sowie besonders bevorzugt mindestens 3 Wochen formstabil sind.

Zur Prüfung der Formstabilität im Sinne der vorliegenden Erfindung werden quaderförmige Prüfkörper des ausgehärteten Dentalabformmaterials aus Komponente A und B mit Abmessung 40 x 12,5 x 5 mm und einer Shore A-Härte im Bereich von 50 - 70 auf einem Objektträger einseitig befestigt, so dass eine Hälfte 20 x 12,5 x 5 mm ungestützt über dem Objektträger hinausragt und der Schwerkraft seines Eigengewichtes ausgesetzt ist. Die unterstützte bzw. die herausragende Fläche des Prüfkörpers wird so gewählt, dass keine spontane Deformation alleine durch die Schwerkraft auftritt. Die hier angegebene Unterstützung auf der Hälfte der Prüfkörperfläche hat sich für Prüfkörper mit einer Shore-Härte von 50 bis 70 bewährt. Weichere Prüfkörper mit einer Shore A-Härte von 30 - 50 sollten mit einer größeren Unterstützungsfläche von 70 % von 40 mm entsprechend 28 x 12,5 x 5 mm und entsprechend kleineren hinausragenden Fläche von 30 % von 40 mm entsprechend 12 x 12,5 x 5 mm aufgebracht werden. In bestimmten Zeitabständen wird die vertikale Auslenkung I (Deformation) des bei der Prüftemperatur von z.B. 23°C oder 60°C gelagerten Prüfkörpers zu dessen ursprünglicher Ausgangsposition gemessen.

Unter formstabil im Sinne der Erfindung sind Prüfkörper zu verstehen, deren Deformation I nach Lagerung für 1 Woche bei 60°C oder für 12 Wochen bei Raumtemperatur weniger als 1 mm, bevorzugt weniger als 0,5 mm, besonders bevorzugt weniger als 0,25 mm und ganz besonders bevorzugt weniger als 0,1 mm betragen. Zudem sollte die Rückstellung nach der Verformung für einen formstabilen Prüfkörper nach Lagerung für 1 Woche bei 60°C oder für 12 Wochen bei Raumtemperatur zum Ausgangswert, gemessen nach ISO 4823 (August 2001) Prüfpunkt 9.7, um weniger als 0,4, bevorzugt um weniger als 0,3, besonders bevorzugt um weniger als 0,2 und insbesondere bevorzugt um weniger als 0,1 abweichen. Die Shore A-Härte für einen formstabilen Prüfkörper nach Lagerung für 1 Woche bei 60°C oder für 12 Wochen bei Raumtemperatur sollte nach DIN 53505 (August 2000) um weniger als 4, bevorzugt um weniger als 3, besonders bevorzugt um weniger als 2 und insbesondere bevorzugt um weniger als 1 gegenüber dem Ausgangswert abnehmen.

Gemäß einer weiteren bevorzugten Ausführungsform der vorliegenden Erfindung ist das Zweikomponeriten-Dentalmaterial so formuliert, dass die den Alkoxysilyl- und/oder Hydroxysilylpolyether a) sowie die wenigstens eine saure Verbindung d) enthaltende Basiskomponente A bei Raumtemperatur für mindestens 6 Monate, bevorzugt mindestens 12 Monate, besonders bevorzugt mindestens 18 Monate und ganz besonders bevorzugt mindestens 24 Monate lagerstabil und/oder bei einer Lagertemperatur zwischen 40°C und 60°C für mindestens 1 Woche, bevorzugt mindestens 2 Wochen, besonders bevorzugt mindestens 3 Wochen und ganz besonders bevorzugt mindestens 4 Wochen lagerstabil ist.

Unter kinetischer Lagerstabilität wird im Sinne der vorliegenden Erfindung verstanden, dass die Komponente A mit dem Zusatz der sauren Verbindung d) keine Vorvernetzung während der Lagerung von 6 Monaten, bevorzugt 12 Monaten und besonders bevorzugt 24 Monaten aufweist. Aufschluss über eine eventuelle Vorvernetzung während der Lagerzeit können rheologische Untersuchungen des Verlust- und des Speichermoduls geben. Für eine kinetische Lagerstabilität darf das Speichermodul einen definierten Grenzwert nicht übersteigen.

Unter Lagerstabilität wird im Sinne der vorliegenden Erfindung verstanden, dass die Basiskomponente A mit dem Zusatz an saurer Verbindung, die rheologischen Eigenschaften beibehält, d. h. dass keine Viskositätsänderung zu höheren oder niedrigen Viskositäten/Konsistenzen hin auftritt, wobei die Konsistenzmessung mit 0,5 ml, 500 g und 15 Sekunden in Anlehnung an ISO 4823 (August 2001), Prüfpunkt 9.2, mit einer Toleranz von ± 4 mm, bevorzugt ± 3 mm, besonders bevorzugt ± 2 mm und insbesondere bevorzugt ± 1 mm erfolgt.

Vorzugsweise ist die Basiskomponente A mit dem Zusatz an saurer Verbindung d) auch hinsichtlich der Reaktionskinetik/Aushärtung lagerstabil, das heißt, nach dem Mischen der Komponenten A und B ist die ursprünglich eingestellte Verarbeitungs- und Aushärtezeit innerhalb bestimmter Toleranzen konstant, wobei die Verarbeitungszeit mit einem McCabe-Rheometer und die Abbindezeit mit einem Brabender Cycloviscograph E ermittelt wird. Bevorzugt liegen die Toleranzen für die Verarbeitungs- und/oder Aushärtezeit bei ± 45 Sekunden, besonders bevorzugt bei ± 30 Sekunden und ganz besonders bevorzugt bei ± 15 Sekunden.

Des weiteren sollte die durch den Zusatz von mindestens einer sauren Verbindung d) verursachte spontane Abbindeverzögerung des Dentalabformmaterials nach dem Mischen der Komponenten A und B innerhalb bestimmter Toleranzen liegen, wobei die Differenz der Abbindezeiten mit und ohne Zusatz von sauren (Neutralisations)mitteln vorzugsweise ± 1,0 Minute, besonders bevorzugt ± 45 Sekunden, ganz besonders bevorzugt ± 30 Sekunden und höchst bevorzugt ± 15 Sekunden beträgt.

Als alkoxysilylfunktionelle und/oder hydroxysilylfunktionelle Polyether a) können prinzipiell alle Polyether enthaltend Alkoxysilylgruppen und/oder Hydroxysilylgruppen eingesetzt werden, wobei das Polyetherrückgrat linear und/oder verzweigt und beispielsweise aus Polyethylenoxid, Polypropylenoxid, Polytetrahydrofuran und/oder deren Copolymeren aufgebaut sein kann, wobei diese Monomere statistisch, blockweise oder in einer taktischen Ordnung vorliegen können. Als Starter für die Polyether und/oder Copolymere können ein- oder mehrwertige Alkohole verwendet werden, wie bspw. Methanol, Butanol, Glycerin, Trimethylpropan, Pentaerythrit und Sorbitol. Beispielsweise können Copolymere aus Polytetrahydrofuran mit Polyethylenoxid oder aus Polyethylenoxid und Polypropylenoxid eingesetzt werden, wobei reines Polypropylenoxid besonders bevorzugt ist. Ferner bevorzugt sind Polyether mit seitenständigen Alkylgruppen, wobei jede oder wenigstens jede zehnte Monomerstruktureinheit eine seitenständige Alkylgruppe trägt. Geeignete Handelsprodukte sind Acclaim 4200, Acclaim 6320N, Acclaim 12200, Acclaim 8200 und Acclaim 6300 der Bayer AG, Polyglycol P41/300 und Polyglycol P41/3000 (Clariant) sowie Poly-(ethylenglycol-ran-propylenglycol) (Aldrich). Bevorzugt weisen die Polyether a) ein zahlengemitteltes Molekulargewicht von 500 bis 25.000 g/mol und besonders bevorzugt von 5.000 bis 20.000 g/mol auf.

Ein Auswahlkriterium für den erfindungsgemäß geeigneten Polyether ist neben der Kettenlänge (Elastizität), der Alkoxysilyl- und/oder Hydroxysilylfunktionalisierung (Aushärte-Kinetik) und der Anzahl der Urethan-/Harnstoffgruppen (Viskosität, Rheologie) die Hydrophilie des Polyethers, die über die Anzahl, Struktur und Polarität der Monomerwiederholungseinheiten des Polyetherpolymers bestimmt wird. Die Hydrophilie für ein Dentalabformmaterial muss einerseits ausreichend hoch sein, um ein gutes Anfließen an feuchte Zahnsubstanz sicherzustellen (niedrige Kontaktwinkel), aber andererseits darf das Material nicht zu hydrophil sein, da sonst Wasser, Feuchtigkeit oder Speichel während der Abdrucknahme bzw. beim Desinfizieren bzw. beim Ausgießen mit Gips zur Quellung führt und damit die erforderliche Dimensionsgenauigkeit nicht mehr gegeben ist. Die Hydrophilie des Polyethers ist darüber hinaus auch unter anderem für die Polyetherlöslichkeit der erfindungsgemäß einzusetzenden sauren Verbindung d) mit verantwortlich.

Gemäß einer bevorzugten Ausführungsform der vorliegenden Erfindung weist der wenigstens eine alkoxysilylfunktionelle und/oder hydroxysilylfunktionelle Polyether einen Gehalt an Polyethergruppen zwischen 5 und 30 mmol/g und besonders bevorzugt zwischen 10 und 25 mmol/g auf.

Vorzugsweise ist die Alkoxysilyl- und/oder Hydroxysilylstruktureinheit bzw. sind die Alkoxysilyl- und/oder Hydroxysilylstruktureinheiten des Polyethers a), bezogen auf das Polymerrückgrat, terminal angeordnet und fallen unter die allgemeine Formel I

- SiR⁵R⁶R⁷

worin R⁵, R⁶ und R⁷ unabhängig voneinander Alkoxy-, Hydroxy-, Alkyl-, Aryl-, Aralkyl-, Alkylarylgruppen, Wasserstoff oder Gruppen der Struktur -(O-CₓH₂ₓ)_{y}-OR mit x = 1 bis 10, bevorzugt x = 1 bis 6, und y = 1 bis 100, bevorzugt y = 2 bis 6, besonders bevorzugt y = 2 bis 4 und insbesondere bevorzugt y = 3, sowie R = H oder Alkyl, mit R⁵, R⁶ und R⁷ unabhängig voneinander bevorzugt Butoxy-, Propoxy-, Ethoxy-, Methoxy-, Hexyl-, Pentyl-, Butyl-, Propyl-, Ethyl- oder Methylgruppen, besonders bevorzugt Hydroxy-, Ethoxy-, Methoxy-, Ethyl- oder Methylgruppen, sind mit der Maßgabe, dass mindestens einer der vorgenannten Reste, vorzugsweise zwei oder alle drei Reste, Alkoxy- und/oder Hydroxygruppen sind.

In Weiterbildung des Erfindungsgedankens wird vorgeschlagen, dass der wenigstens eine Polyether a) einen Alkoxy- und/oder Hydroxygruppengehalt von 0,02 bis 12 mmol/g, besonders bevorzugt von 0,04 bis 6 und ganz besonders bevorzugt von 0,04 bis 3 mmol/g, aufweist.

Durch die Art und Anzahl der Alkoxy- und/oder Hydroxygruppen pro Siliziumatom lässt sich die Kondensationskinetik und damit der Verarbeitungs- und Abbindezeit des Dentalmaterials einstellen. Bevorzugt werden diese Parameter derart gewählt, dass die Verarbeitungszeit 30 Sekunden bis 3 Minuten, besonders bevorzugt zwischen 1 und 2,5 Minuten und ganz besonders bevorzugt zwischen 1,5 und 2 Minuten und/oder die nach ISO 4823 (Ausgabe 1992) bestimmte Abbindezeit im Patientenmund (sog. Mundverweildauer) maximal 15 Minuten, besonders bevorzugt maximal 10 Minuten, ganz besonders bevorzugt maximal 7 Minuten und höchst bevorzugt kleiner gleich 6 Minuten beträgt.

Vorzugsweise weist der wenigstens eine Polyether a) als dritte Struktureinheit (neben den terminalen Alkoxy- und/oder Hydroxygruppen und den Polyethergruppen) jeweils an den terminalen Alkoxysilyl- und/oder Hydroxysilylgruppen angeordnete Alkylenspacer, welche bevorzugt C₁-C₆-Alkylgruppen, besonders bevorzugt C₁-C₃-Alkylgruppen, ganz besonders bevorzugt Ethylengruppen und/oder Methylengruppen und höchst bevorzugt Methylengruppen sind, auf.

Zudem kann der wenigstens eine Polyether a) als vierte Struktureinheit 0 bis 8 mmol/g, besonders bevorzugt 0 bis 4 mmol/g, ganz besonders bevorzugt 0,02 bis 2 mmol/g und höchst bevorzugt 0,1 bis 0,4 mmol/g Urethangruppen und/oder 0 bis 8 mmol/g, besonders bevorzugt 0 bis 2 mmol/g, ganz besonders bevorzugt 0,02 bis 2 mmol/g und höchst bevorzugt 0,1 bis 0,4 mmol/g Harnstoffgruppen aufweisen. Insbesondere dann, wenn der wenigstens eine Polyether a) als vierte Struktureinheit Harnstoff- und/oder Urethangruppen aufweist, ist eine Methylengruppe als Spacer bevorzugt. Durch den Einsatz solcher α-aktivierter Alkoxysilyl- und/oder Hydroxysilylpolyether werden hydrophile, lagerstabile Zweikomponenten-Dentalabformmassen erhalten, welche mit einem als Katalysator einzusetzenden Salz oder Base überraschend schnell durch Kondensationsreaktion vernetzen. Insgesamt sollte der Gehalt an Urethan- bzw. Harnstoffgruppen pro Molekül so gering wie möglich gehalten werden, um die intermolekularen Wechselwirkungen zwischen den einzelnen Polyetherketten zu minimieren, um die durch den Polyetherzusatz begründete Viskosität so gering wie möglich zu halten, was den Zusatz höherer Mengen an Füllstoffen in dem Dentalmaterial ermöglicht und somit mehr Formulierungsfreiräume und kostengünstigere Rezepturen einräumt.

Im Rahmen der vorliegenden Erfindung hat es sich als vorteilhaft erwiesen, Polyether, die innerhalb der Polymerkette keine Urethan- oder Harnstoffgruppen enthalten und die an jedem Kettenende höchstens/nicht mehr als eine Urethan- oder Harnstoffgruppe und höchstens/nicht mehr als eine Alkoxy- und/oder Hydroxysilylgruppe und höchstens/nicht mehr als eine Methylenspacergruppe tragen, einzusetzen. Der Einsatz dieser Polyether führt verglichen mit den im Stand der Technik eingesetzten Polyurethan-Alkoxysilyl- und/oder Hydroxysilylpolyethern zu Formulierungen mit niedrigerer Viskosität, so dass den Dentalmaterialien mehr Füllstoffe zugegeben werden können, was zu einer Reduzierung der Herstellkosten führt.

Gemäß einer weiteren besonderen Ausführungsform der vorliegenden Erfindung sind die einzelnen Struktureinheiten des wenigstens einen Polyethers a) folgendermaßen angeordnet: 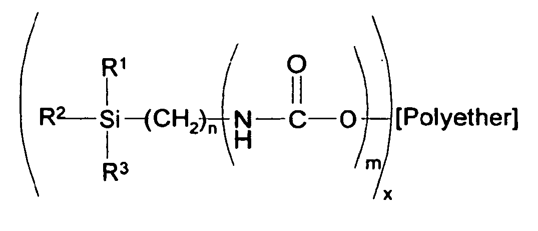 worin R¹, R² und R³ unabhängig voneinander Alkoxy-, Hydroxy-, Alkyl-, Aryl-, Aralkyl-, Alkylarylgruppen, Wasserstoff oder Gruppen der Struktur -(O-CₓH₂ₓ)_{y}-OR mit x = 1 bis 10, bevorzugt x = 1 bis 6, und y = 1 bis 100, bevorzugt y = 2 bis 6, besonders bevorzugt y = 2 bis 4 und insbesondere bevorzugt y = 3, sowie R = H oder Alkyl, mit R¹, R² und R³ unabhängig voneinander bevorzugt Butoxy-, Propoxy-, Ethoxy-, Methoxy-, Hexyl-, Pentyl-, Butyl-, Propyl-, Ethyl- oder Methylgruppen, besonders bevorzugt Hydroxy-, Ethoxy-, Methoxy-, Ethyl- oder Methylgruppen, sind mit der Maßgabe, dass mindestens einer der vorgenannten Reste, vorzugsweise zwei oder alle drei Reste, Alkoxy- und/oder Hydroxygruppen sind, sowie x=1 bis 6, bevorzugt x=2 bis 4 und ganz besonders bevorzugt x=2, n=1 bis 6, bevorzugt n=1 bis 3 und ganz besonders bevorzugt n=1 sowie m=0 oder 1, besonders bevorzugt m=1, ist
oder 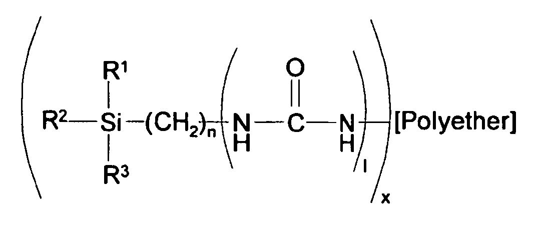 worin R¹, R² und R³ unabhängig voneinander Alkoxy-, Hydroxy-, Alkyl-, Aryl-, Aralkyl-, Alkylarylgruppen, Wasserstoff oder Gruppen der Struktur -(O-CₓH₂ₓ)_{y}-OR mit x = 1 bis 10, bevorzugt x = 1 bis 6, und y = 1 bis 100, bevorzugt y = 2 bis 6, besonders bevorzugt y = 2 bis 4 und insbesondere bevorzugt y = 3, sowie R = H oder Alkyl, mit R¹, R² und R³ unabhängig voneinander bevorzugt Butoxy-, Propoxy-, Ethoxy-, Methoxy-, Hexyl-, Pentyl-, Butyl-, Propyl-, Ethyl- oder Methylgruppen, besonders bevorzugt Hydroxy-, Ethoxy-, Methoxy-, Ethyl- oder Methylgruppen, sind mit der Maßgabe, dass mindestens einer der vorgenannten Reste, vorzugsweise zwei oder alle drei Reste, Alkoxy- und/oder Hydroxygruppen sind, sowie x=1 bis 6, bevorzugt x=2 bis 4 und ganz besonders bevorzugt x=2, n=1 bis 6, bevorzugt n=1 bis 3 und ganz besonders bevorzugt n=1 sowie I=0 oder 1, besonders bevorzugt I=1 ist.

Gemäß einer weiteren bevorzugten Ausführung der vorliegenden Erfindung ist der Alkylspacer in den vorgenannten Struktureinheiten Methylen (n=1).

Die Herstellung dieser alkoxysilylfunktionellen und/oder hydroxysilylfunktionellen Polyether ist bekannt und wird beispielsweise in der DE 101 04 079 A1, EP 0 629 819 B1, DE 101 39 132, US 4,906,707, EP 0 372 561 A1, EP 1 303 560 A1 und EP 0 170 865 B1 beschrieben. Beispiele für kommerziell erhältliche und im Rahmen der vorliegenden Erfindung geeignete Polyether sind MS Polymer S 203H, MS Polymer S 303H (Kaneka), Polymer XP ST55, ST50, ST51, ST53 (Hanse), SLM 414000 (Wacker), SLM 414001 (Wacker), Baycoll XP 2458 und Desmoseal XP 2447 (Bayer AG), wobei der unter dem Handelsnamen SLM 414000 vertriebene Dimethoxy(methyl)silylmethylcarbamat-terminierte Polyether: 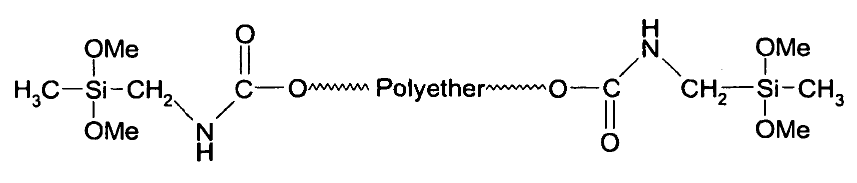 und Dimethoxy(methyl)silylmethylharnstoff-terminierte Polyether: 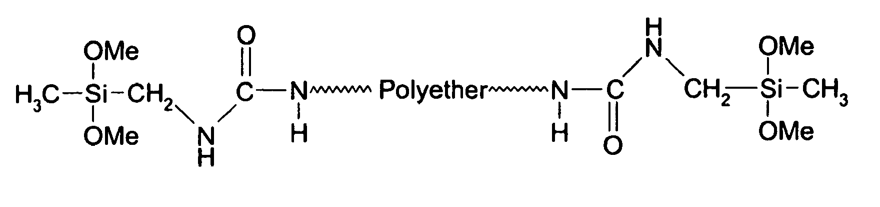 besonders bevorzugt sind.

Grundsätzlich können die erfindungsgemäßen Dentalmaterialien als Katalysator b) alle katalytisch wirksamen freien Basen oder Salze enthalten.

Gemäß einer ersten besonderen Ausführungsform der vorliegenden Erfindung enthält das Dentalmaterial als Katalysator b) eine oder mehrere freie Basen, wobei organische Basen mit einem in Acetonitril gemessenen pK_{BH+}-Wert von mindestens 20 bevorzugt sind.

Dabei weist die erfindungsgemäß als Katalysator einzusetzende wenigstens eine Base b) vorzugsweise einen in Acetonitril gemessenen pK_{BH+}-Wert von mindestens 21, besonders bevorzugt von mindestens 22 und ganz besonders bevorzugt von mindestens 23 auf.

Gemäß einer Unterausführungsform der ersten besonderen Ausführungsform der vorliegenden Erfindung wird als Katalysator b) eine organische Base eingesetzt, welche wenigstens eine Struktureinheit gemäß der allgemeinen Formel 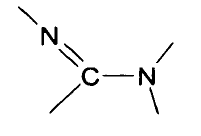 und/oder gemäß der allgemeinen Formel III 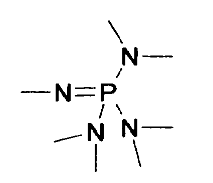 und/oder gemäß der allgemeinen Formel IV 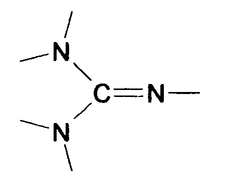 umfasst.

Insbesondere werden gute Ergebnisse erzielt, wenn der Katalysator b) wenigstens eine aus der aus 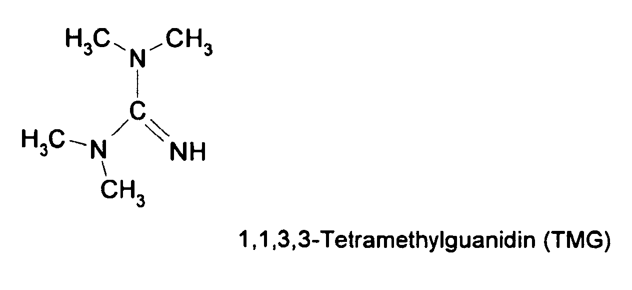 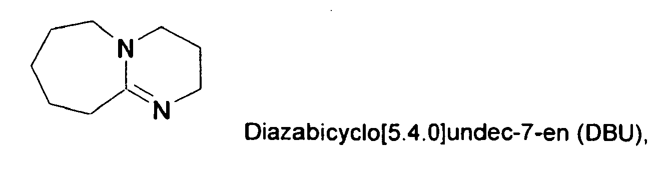 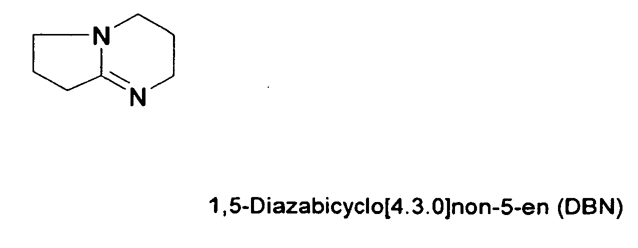 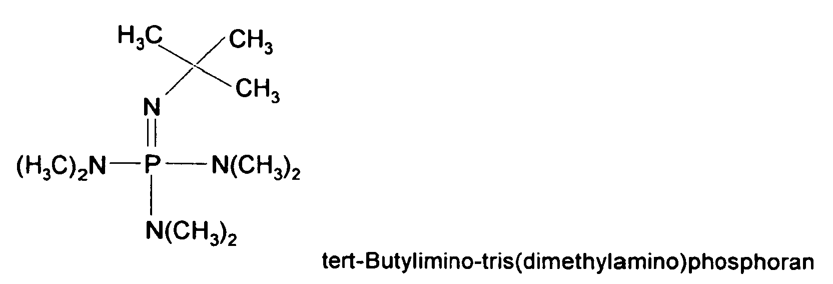 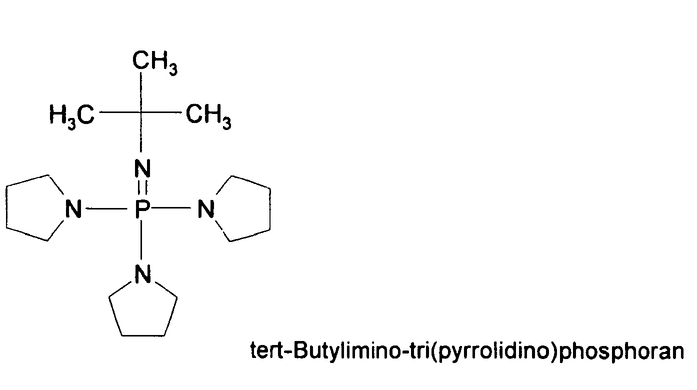 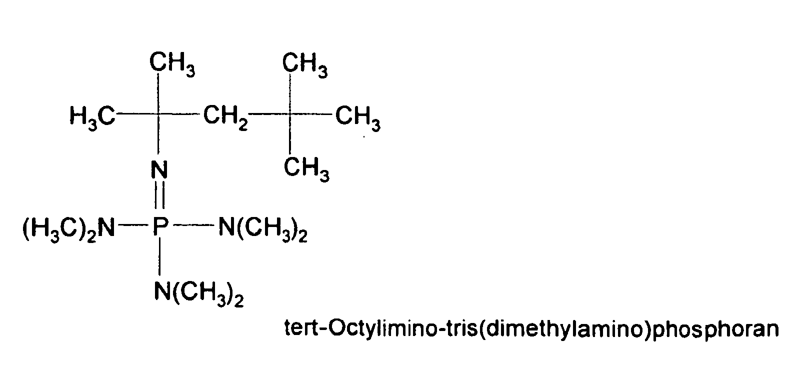 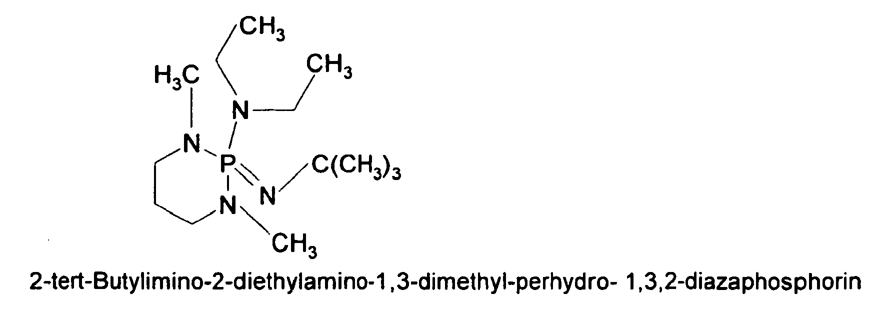 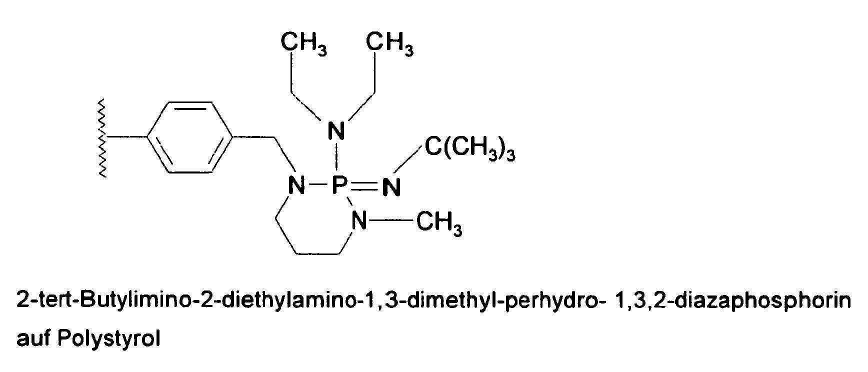 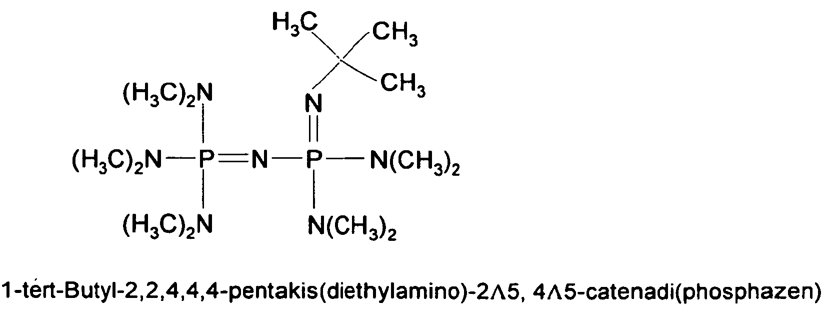 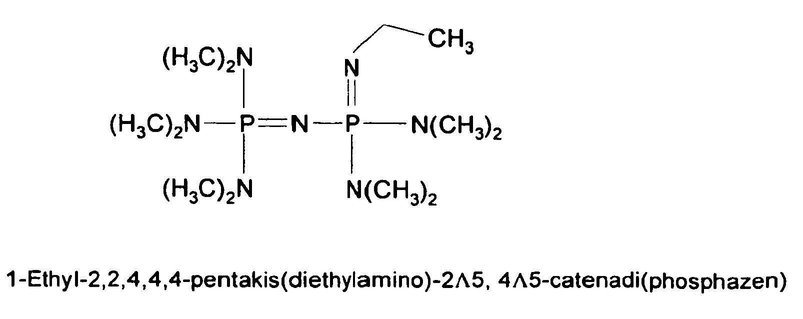 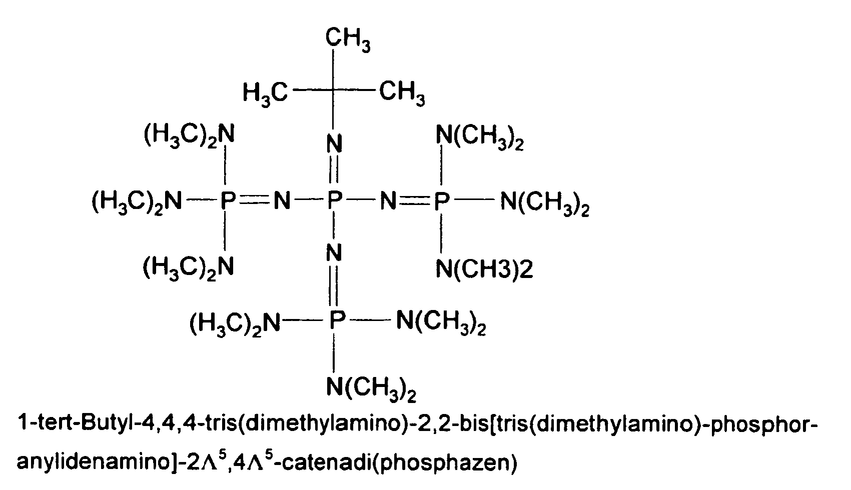 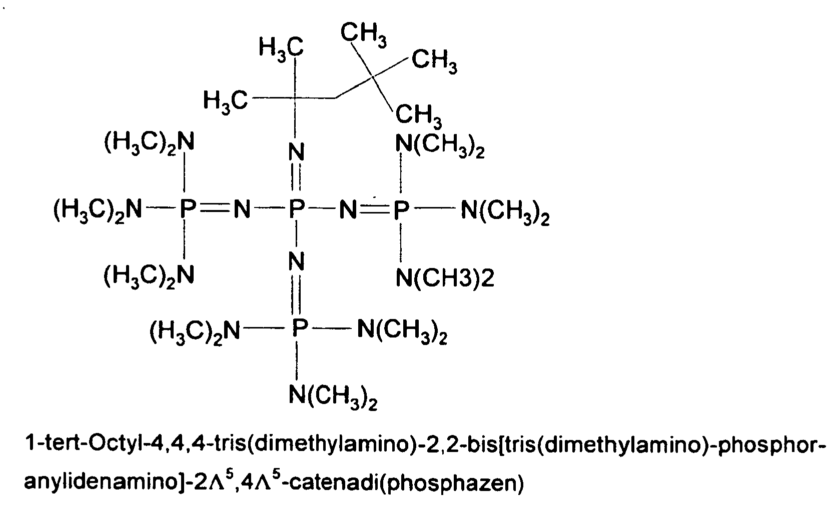 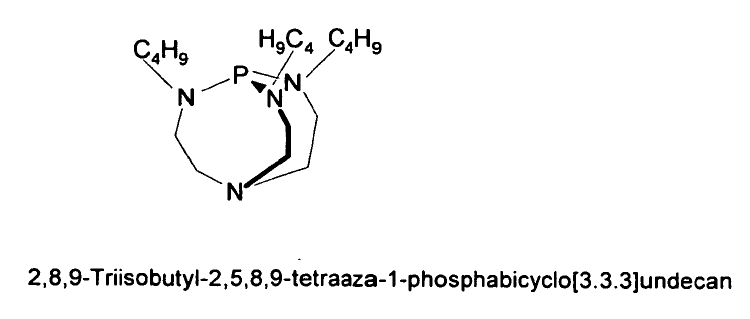 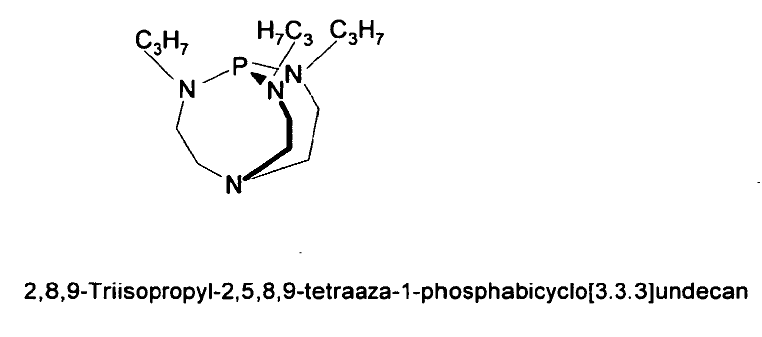 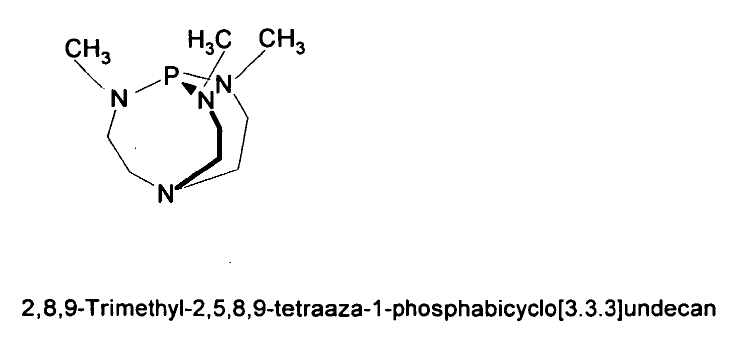 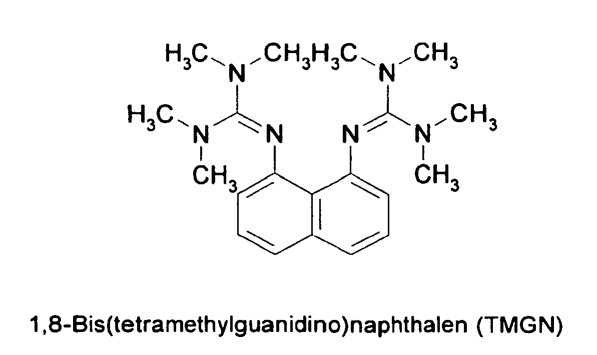 sowie 2-tert-Butyl-1,1,3,3-tetramethylguanidin, 1,5,7-Triazabicyclo(4.4.0)dec-5-en, 7-Methyl-1,5,7-triazabicyclo(4.4.0)dec-5-en, 1,5-Diazabicyclo[4.3.0)non-5-en und 3,3,6,9,9-Pentamethyl-2,10-diazabicyclo-(4.4.0)dec-1-en bestehenden Gruppe ausgewählte organische Base ist.

Ganz besonders bevorzugt enthält das erfindungsgemäße Dentalmaterial als Katalysator b) wenigstens eine aus der aus tert-Butylimino-tri(pyrrolidino)phosphoran, 1-tert-Butyl-2,2,4,4,4-pentakis(diethylamino)-2A5, 4A5-catenadi(phosphazen), 1-tert-Butyl-4,4,4-tris(dimethylamino)-2,2-bis[tris-(dimethylamino)-phosphoranylidenamino]-2Λ⁵,4Λ⁵-catenadi(phosphazen), tert-Octylimino-tris(dimethylamino)phosphoran, 2,8,9-Tri-isopropyl-2,5,8,9-tetraaza-1-phosphabicyclo[3.3.3]undecan, 1,5-Diazabicyclo[4.3.0]non-5-en, 1,1,3,3-Tetramethylguanidin, Diazabicyclo[5.4.0]undec-7-en, 7-Methyl-1,5,7-triazabicyclo(4.4.0)dec-5-en, 2-tert-Butyl-1, 1,3,3-tetra-methylguanidin, 1,5,7-Triazabicyclo(4.4.0)dec-5-en und 1,8-Bis(tetramethylguanidino)naphthalen bestehenden Gruppe ausgewählte Base.

In Weiterbildung des Erfindungsgedankens wird vorgeschlagen, dass die vorgenannten, erfindungsgemäß als Katalysator b) einzusetzenden organischen Basen Alkoxysilylgruppen aufweisen. Dadurch wird erreicht, dass die Base nach Aushärten des Dentalmaterials in der Polyethermatrix eingebunden ist und aus dem dentalen Abformmaterial nicht mehr herausgelöst werden kann.

Erfindungsgemäß können die Dentalmaterialien als Katalysator b) eine oder, in beliebiger Kombination miteinander, mehrere der zuvor genannten organischen Basen enthalten. Vorzugsweise enthält das Dentalmaterial nur eine der zuvor genannten organischen Basen als Katalysator b). Besonders bevorzugt werden neben der einen oder mehreren erfindungsgemäß einzusetzenden organischen Basen keine weiteren Katalysatoren, insbesondere keine metallorganischen Metallsalze, keine Amine mit pk_{BH+}-Werten < 20 und keine freien Säuren, eingesetzt.

Wie der Fachmann erkennt, hängt die Menge an einzusetzender Katalysatorbase u.a. von der Löslichkeit der Base in der eingesetzten Polyethermatrix ab. Vorzugsweise beträgt die Menge der Katalysatorbase(n), bezogen auf die Gesamtmischung des Dentalmaterials, 0,001 bis 1 mmol/g, bevorzugt 0,001 bis 0,5 mmol/g, ganz besonders bevorzugt 0,001 bis 0,1 mmol/g und höchst bevorzugt 0,005 bis 0,05 mmol/g. Selbstverständlich muss die als Katalysator b) eingesetzte organische Base bzw. die Basenmischung eine Mindestlöslichkeit in der eingesetzten Polyethermatrix aufweisen, um überhaupt katalytisch wirken zu können.

Um die Menge an einzusetzender Katalysatorbase möglichst gering zu halten, wird in Weiterbildung des Erfindungsgedankens vorgeschlagen, in dem Dentalmaterial eine Base mit einer hinreichend hohen Löslichkeit in dem Polyethermaterial, d.h. mit einer ausreichenden katalytischen Aktivität, einzusetzen, wobei die katalytische Aktivität im Sinne der vorliegenden Patentanmeldung durch die Aushärtezeit nach ISO 4823 (Ausgabe 1992), bestimmt durch Rückstellung nach der Verformung, charakterisiert ist. Vorzugsweise wird eine Base eingesetzt, die mit Polytetrahydrofuran, Polyethylenglycol und besonders bevorzugt Polypropylenglycol sowie deren Mischungen und Copolymerisaten als Polyethermatrix eine Aushärtezeit kleiner gleich 30 Min., bevorzugt kleiner gleich 15 Min. für eine dentale Zahntechnik-Dubliermasse bzw. eine Aushärtezeit kleiner gleich 15 Min., bevorzugt kleiner gleich 10 Min., besonders bevorzugt kleiner gleich 7 Min. und höchst bevorzugt kleiner gleich 6 Min. für eine dentale Abformmasse ergibt.

Gemäß einer zweiten besonderen Ausführungsform der vorliegenden Erfindung enthält das Zweikomponenten-Dentalmaterial als Katalysator b) ein Salz ist, welches gebildet ist aus wenigstens einem aus der aus
- Komplexen von Alkalimetall- oder Ammonium-Kationen mit Kronenethern und/oder Kryptanden,
- Tetraalkyl-, Tetraaryl-, Trialkylaryl-, Dialkyldiaryl-, Monoalkyltriarylammonium-Kationen, Tetraalkyl-, Tetraaryl-, Trialkylaryl-, Dialkyldiaryl-, Monoalkyltriarylphosphonium-Kationen, Tetraalkyl-, Tetraaryl-, Trialkylaryl-, Dialkyldiaryl-, Monoalkyltriarylarsonium-Kationen, Tetraalkyl-, Tetraaryl-, Trialkylaryl-, Dialkyldiaryl-, Monoalkyltriarylstibonium-Kationen,
- durch Protonierung einer Base mit einem in Acetonitril gemessenen pK_{BH+}-Wert von mindestens 20 gebildeten Kationen
und beliebigen Kombinationen hiervon bestehenden Gruppe ausgewählten Kation und wenigstens einem Anion einer gesättigten und/oder ungesättigten (cyclo)aliphatischen Carbonsäure, wobei die Carbonsäure eine verzweigte Carbonsäure mit einer Länge der an der Carboxylgruppe vorgesehenen (Cyclo)Alkylkette von wenigstens 2 Kohlenstoffatomen oder eine unverzweigte Carbonsäure mit einer Länge der an der Carboxylgruppe vorgesehenen (Cyclo)Alkylkette von wenigstens 4 Kohlenstoffatomen ist.

Gemäß einer ersten bevorzugten Unterausführungsform der zweiten besonderen Ausführungsform der vorliegenden Erfindung wird als Katalysator b) wenigstens ein Salz gebildet aus wenigstens einem Kronenether-Alkalimetallion-Komplex, Kronenether-Ammoniumion-Komplex, Kryptanden-Alkalimetallion-Komplex und/oder Kryptanden-Ammoniumion-Komplex sowie wenigstens einem Anion einer gesättigten und/oder ungesättigten (cyclo)aliphatischen Carbonsäure, wobei die Carbonsäure eine verzweigte Carbonsäure mit einer Länge der an der Carboxylgruppe vorgesehenen (Cyclo)Alkylkette von wenigstens 2 Kohlenstoffatomen oder eine unverzweigte Carbonsäure mit einer Länge der an der Carboxylgruppe vorgesehenen (Cyclo)Alkylkette von wenigstens 4 Kohlenstoffatomen ist, eingesetzt.

Vorzugsweise wird in dieser Ausführungsform als Kation des Katalysatorsalzes ein Komplex gebildet aus einem oder mehreren Lithium-, Natrium-, Kalium-, Rubidium-, Cäsium- und/oder Ammonium-lonen und einem oder mehreren der nachfolgenden Kronenether und/oder Kryptanden eingesetzt:

15-Crown-5, 18-Crown-6, Dibenzo-18-crown-6, Dibenzo-21-crown-7, Dibenzo-24-crown-8, Dibenzo-30-crown-10, 1,4,10-Trioxa-7,13-diazacyclopentadecan, 4,7,13,18-Tetraoxa-1,10-diazabicyclo[8.5.5]eicosan, 1,4,10,13-Tetraoxa-7,16-diazacyclooctadecan, 3,6,9,14-Tetrathiabicyclo[9.2.1]tetradeca-11,13-dien, 1,4,7,10-Tetrathiacyclododecan, 1,5,9,13-Tetrathiacyclohexadecan-3,11-diol, 1,5,9-Triazacyclododecan, 1,4,7-Triazacyclononan, 1,4,7,10,13,16-Hexamethyl-1,4,7,10,13,16-hexaazacyclooctadecan,

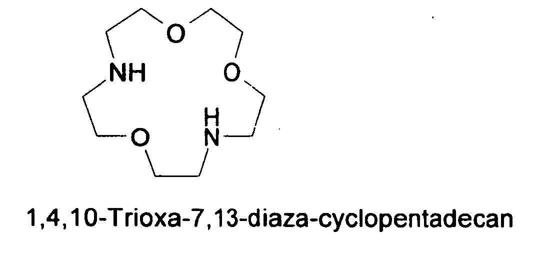

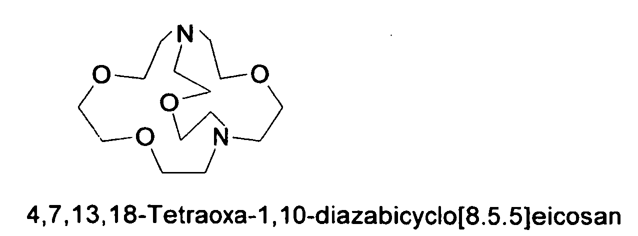

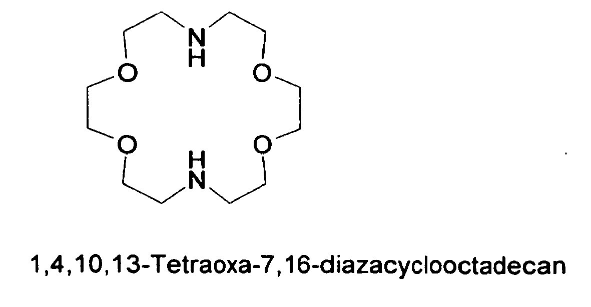

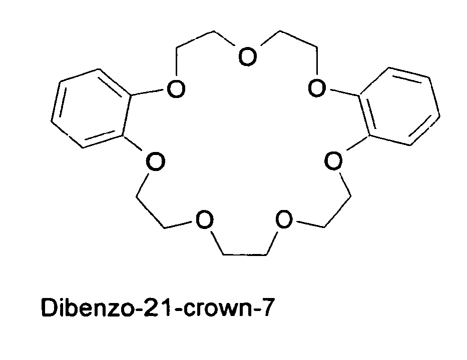

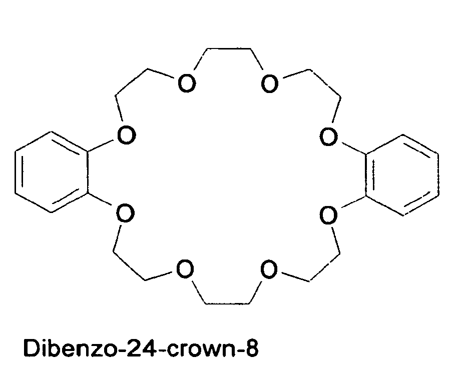

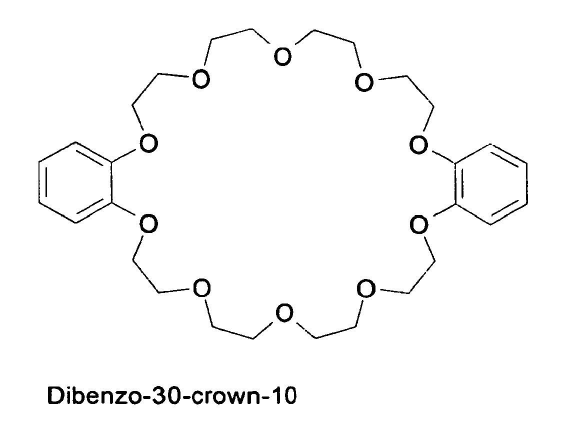

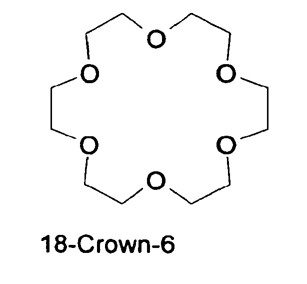

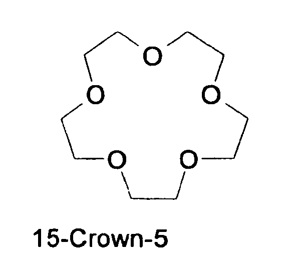

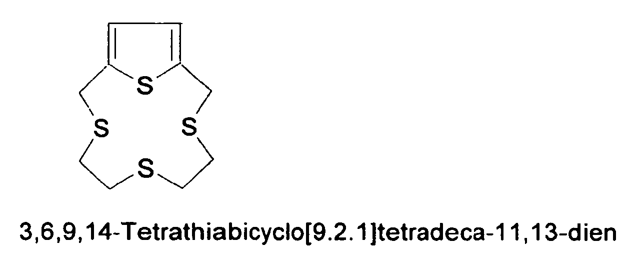

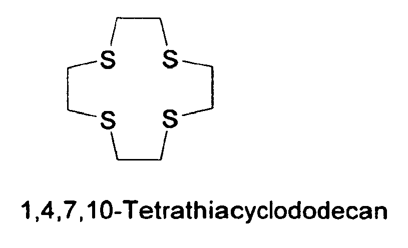

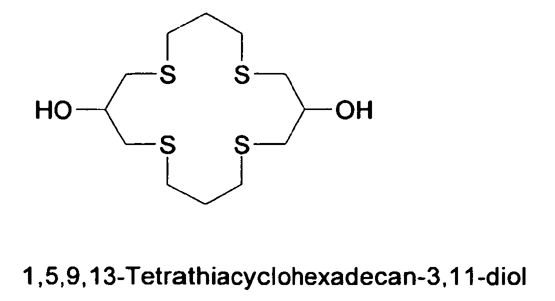

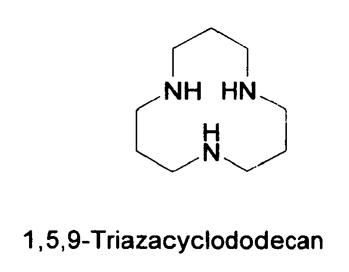

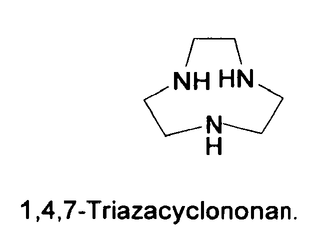

Die Herstellung des in dieser Ausführungsform erfindungsgemäß einzusetzenden Katalysatorsalzes kann auf jede dem Fachmann bekannte Art erfolgen, beispielsweise durch folgende Reaktion:

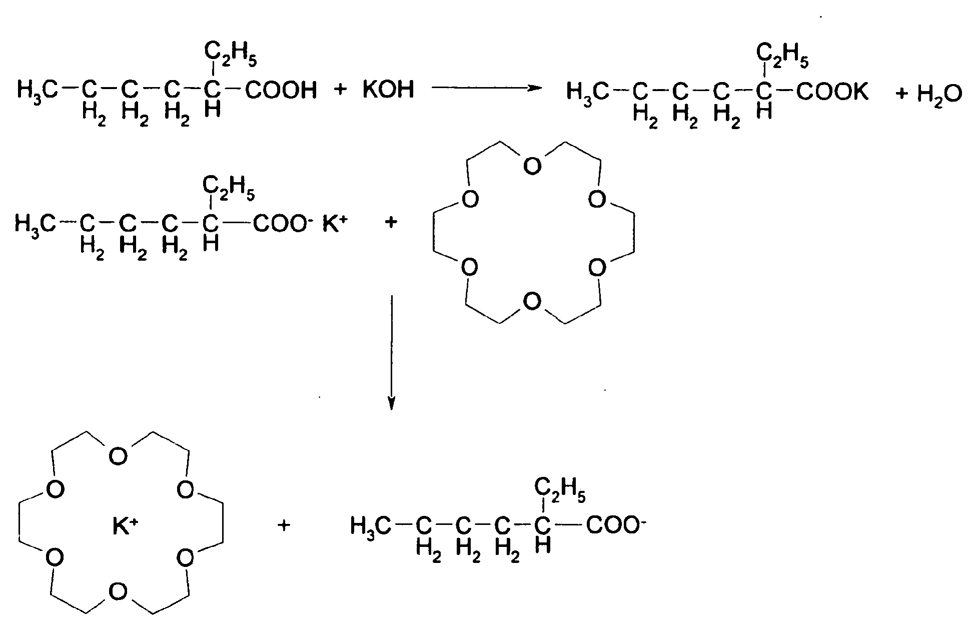

Gemäß einer zweiten bevorzugten Unterausführungsform der zweiten besonderen Ausführungsform der vorliegenden Erfindung wird als Katalysator b) wenigstens ein Salz gebildet aus wenigstens einem Anion einer gesättigten und/oder ungesättigten (cyclo)aliphatischen Carbonsäure, wobei die Carbonsäure eine verzweigte Carbonsäure mit einer Länge der an der Carboxylgruppe vorgesehenen (Cyclo)Alkylkette von wenigstens 2 Kohlenstoffatomen oder eine unverzweigte Carbonsäure mit einer Länge der an der Carboxylgruppe vorgesehenen (Cyclo)Alkylkette von wenigstens 4 Kohlenstoffatomen ist, und einem Tetraalkyl-, Tetraaryl-, Trialkylaryl-, Dialkyldiaryl-, Monoalkyltriarylammonium-, Tetraalkyl-, Tetraaryl-, Trialkylaryl-, Dialkyldiaryl-, Monoalkyltriarylphosphonium-, Tetraalkyl-, Tetraaryl-, Trialkylaryl-, Dialkyldiaryl-, Monoalkyltriarylarsonium-, Tetraalkyl-, Tetraaryl-, Trialkylaryl-, Dialkyldiaryl-, und/oder Monoalkyltriarylstibonium-Kation eingesetzt. Beispiele für geeignete Kationen für die gemäß dieser Ausführungsform einzusetzenden Katalysatorsalze sind Tetramethylammonium-, Tetraethylammonium-, Tetrapropylammonium-, Tetrabutylammonium-, Tetrapentylammonium-, Tetrahexylammonium-, Tetraheptylammonium-, Tetraoctylammonium-, Tetranonylammonium-, Tetradecylammonium-, Tetramethylphosphonium-, Tetraethylphosphonium-, Tetrapropylphosphonium-, Tetrabutylphosphonium-, Tetrapentylphosphonium-, Tetrahexylphosphonium-, Tetraheptylphosphonium-, Tetraoctylphosphonium-, Tetranonylphosphonium-, Tetradecylphosphonium-, Tetramethylarsonium-, Tetraethylarsonium-, Tetrapropylarsonium-, Tetrabutylarsonium-, Tetrapentylarsonium-, Tetrahexylarsonium-, Tetraheptylarsonium-, Tetraoctylarsonium-, Tetranonylarsonium-, Tetradecylarsonium-, Tetramethylstibonium-, Tetraethylstibonium-, Tetrapropylstibonium-, Tetrabutylstibonium-, Tetrapentylstibonium-, Tetrahexylstibonium-, Tetraheptylstibonium-, Tetraoctylstibonium-, Tetranonylstibonium-, Tetradecylstibonium-, Lauryltrimethylammonium-, Myristyltrimethylammonium-, Cetyltrimethylammonium-, Stearyltrimethylammonium-, Lauralkonium-, Myristalkonium-, Cetalkonium-, Stearalkonium-, Cetylethyldimethylammonium-, Benzyltriethylammonium- und Benzalkonium-lonen.

Die Herstellung der Salze aus den vorgenannten Carbonsäureanionen und den alkyl- und/oder arylgruppensubstituierten Ammonium-/Phosphonium-/ Arsonium-/Stiboniumionen gemäß dieser Ausführungsform kann auf jede dem Fachmann bekannte Weise erfolgen, wobei sich beispielsweise die folgender Synthese als besonders geeignet erwiesen hat:

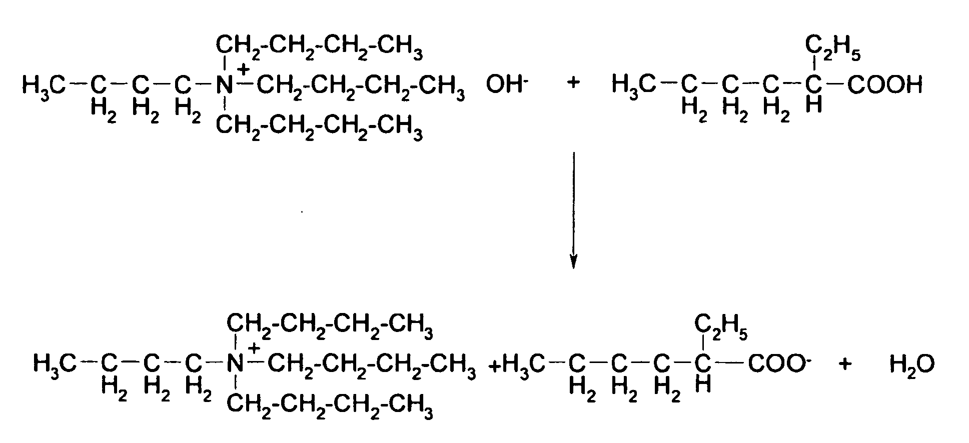

Gemäß einer dritten bevorzugten Unterausführungsform der zweiten besonderen Ausführungsform der vorliegenden Erfindung wird als Katalysator b) wenigstens ein Salz gebildet aus wenigstens einem Anion einer gesättigten und/oder ungesättigten (cyclo)aliphatischen Carbonsäure, wobei die Carbonsäure eine verzweigte Carbonsäure mit einer Länge der an der Carboxylgruppe vorgesehenen (Cyclo)Alkylkette von wenigstens 2 Kohlenstoffatomen oder eine unverzweigte Carbonsäure mit einer Länge der an der Carboxylgruppe vorgesehenen (Cyclo)Alkylkette von wenigstens 4 Kohlenstoffatomen ist, und einem durch Protonierung einer Base mit einem in Acetonitril gemessenen pK_{BH+}-Wert von mindestens 20 gebildeten Kation eingesetzt. Besonders gute Ergebnisse werden erhalten, wenn das Dentalmaterial als Katalysator b) ein aus einem vorgenannten Anion und einem durch Protonierung einer Base mit einem in Acetonitril gemessenen pK_{BH+}-Wert von mindestens 21, besonders bevorzugt mindestens 22 und ganz besonders bevorzugt mindestens 23 gebildeten Kation gebildetes Salz enthält.

In Weiterbildung des Erfindungsgedankens wird vorgeschlagen, in der dritten Unterausführungsform der vorliegenden Erfindung als Katalysator b) ein aus einem Anion einer der vorgenannten Carbonsäuren und einer protonierten Base gebildetes Salz einzusetzen, wobei die Base eine Struktur aufweist, welche nach Protonierung der Base eine Mesomeriestabilisierung der positiven Ladung ermöglicht. Mesomeriestabilisierung im Sinne der vorliegenden Erfindung bedeutet im Einklang mit dem allgemeinen Lehrbuchwissen, dass sich für die protonierte Base wenigstens zwei Grenzstrukturen formulieren lassen, in denen die positive Ladung an unterschiedlichen Atomen lokalisiert ist, bzw., dass in der protonierten Base π-Elektronen delokalisiert sind. Bevorzugt sind insbesondere aus einem Anion einer der vorgenannten Carbonsäuren und einer protonierten Base gebildete Katalysatorsalze, wobei die Base wenigstens eine Struktureinheit gemäß einer der vorgenannten allgemeinen Formeln II bis IV aufweist. Diese Struktureinheiten führen nach der Protonierung der Base zu einer guten Mesomeriestabilisierung der positiven Ladung, was zu einer Stabilisierung der protonierten Form führt.

Insbesondere werden für die dritte Unterausführungsform der zweiten besonderen Ausführungsform der vorliegenden Erfindung gute Ergebnisse erzielt, wenn als Kation eine protonierte Base eingesetzt wird, welche aus der für die erste besondere Ausführungsform der vorliegenden Erfindung beschriebenen Gruppe ausgewählt ist.

Bevorzugt ist die Basenkomponente des Salzes gemäß dieser Ausführungsform der vorliegenden Erfindung tert-Butylimino-tri(pyrrolidino)phosphoran, 1-tert-Butyl-2,2,4,4,4-pentakis(diethylamino)-2A5, 4A5-catenadi(phosphazen), 1-tert-Butyl-4,4,4-tris(dimethylamino)-2,2-bis[tris(dimethylamino)-phosphoranylidenamino]-2A⁵,4A⁵-catenadi(phosphazen), tert-Octylimino-tris(dimethylamino)-phosphoran, 2,8,9-Tri-isopropyl-2,5,8,9-tetraaza-1-phosphabicyclo-[3.3.3]undecan, 1,5-Diazabicyclo[4.3.0]non-5-en, 1,1,3,3-Tetramethylguanidin, Diazabicyclo[5.4.0]undec-7-en, 7-Methyl-1,5,7-triazabicyclo(4.4.0)dec-5-en, 2-tert-Butyl-1,1,3,3-tetra-methylguanidin, 1,5,7-Triazabicyclo(4.4.0)dec-5-en und/oder 1,8-Bis(tetramethylguanidino)naphthalen.

Die Herstellung des durch Säure-Base-Reaktion gebildeten Katalysatorsalzes gemäß der dritten Ausführungsform der vorliegenden Erfindung kann auf jede dem Fachmann bekannte Weise erfolgen, beispielsweise durch die nachfolgende Reaktion:

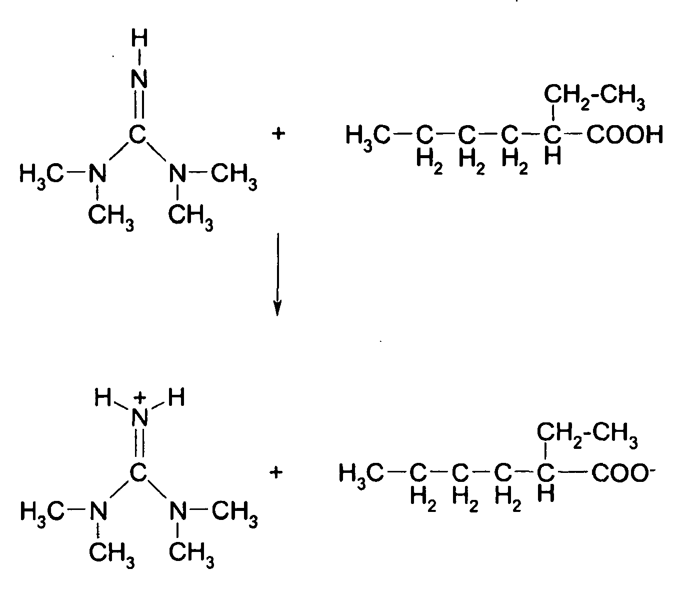

Erfindungsgemäß können in den Zweikomponenten-Dentalmaterialien als Katalysator alle aus wenigstens einem der vorgenannten Kationen und wenigstens einem der vorgenannten Carbonsäureanionen gebildeten Salze eingesetzt werden, wobei insbesondere Salze, in denen das Anion des Katalysatorsalzes ein Anion einer verzweigten Carbonsäure mit einer Länge der an der Carboxylgruppe vorgesehenen (Cyclo)Alkylkette von wenigstens 3 Kohlenstoffatomen, besonders bevorzugt wenigstens 4 und ganz besonders bevorzugt wenigstens 5 Kohlenstoffatomen oder einer unverzweigten Carbonsäure mit einer Länge der an der Carboxylgruppe vorgesehenen (Cyclo)Alkylkette von wenigstens 5 Kohlenstoffatomen ist, bevorzugt sind. Des weiteren bevorzugt sind Salze, in denen das Anion ein

Anion einer gesättigten und/oder ungesättigten (cyclo)aliphatischen Monocarbonsäure mit einer der zuvor genannten (Cyclo)Alkylkettenlänge ist.

Besonders gute Ergebnisse werden insbesondere auch mit Salzen umfassend wenigstens ein durch Deprotonierung resultierendes aliphatisches oder cycloaliphatisches Carbonsäureanion mit einer wenigstens eine und bevorzugt wenigstens zwei Verzweigungen aufweisenden (Cyclo)Alkylkette erzielt. Vorzugsweise wird als Anion des wenigstens einen Katalysatorsalzes ein aliphatisches oder cycloaliphatisches Carbonsäureanion eingesetzt, das wenigstens eine Verzweigung in γ-Stellung, besonders bevorzugt wenigstens eine Verzweigung β-Stellung und ganz besonders bevorzugt wenigstens eine Verzweigung in α-Stellung zur Carboxylgruppe des Carbonsäureanions aufweist. Ebenso bevorzugt sind entsprechende Carbonsäureanionen, in deren (Cyclo)Alkylkette sowohl in γ-, β- und/oder α-Stellung zu der Carboxylgruppe des Carbonsäureanions jeweils wenigstens eine Verzweigung vorgesehen ist.

In Weiterbildung des Erfindungsgedankens wird insbesondere für die dritte Unterausführungsform, in der das Kation des Katalysatorsalzes durch Protonierung einer Base gebildet wird, vorgeschlagen, in dem Dentalmaterial als Katalysator b) wenigstens ein Salz gebildet aus einer der vorgenannten Kationen und einem Anion einer aus der aus 2,2-Dialkylalkansäure, 3,3-Dialkylalkansäure, 4,4-Di-alkylalkansäure, 2,3-Dialkylalkansäure, 2,4-Dialkylalkansäure, 3,4-Dialkylalkansäure, 2,2-Dialkylalkensäure, 3,3-Dialkylalkensäure, 4,4-Dialkylalkensäure, 2,3-Dialkylalkensäure, 2,4-Dialkylalkensäure, 3,4-Dialkylalkensäure, 2,2-Dialkylalkinsäure, 3,3-Dialkylalkinsäure, 4,4-Dialkylalkinsäure, 2,3-Dialkylalkinsäure, 2,4-Dialkylalkinsäure, 3,4-Dialkylalkinsäure, 2-Monoalkylalkansäure, 3-Monoalkylalkansäure, 4-Monoalkylalkansäure, 2,2-Dialkylhexansäure, bevorzugt 2,2-Dialkylnonan-säure, 2,2-Dimethyldecansäure, 2,2-Diethyldecansäure, 2,2-Dipropyldecan-säure, 2,2-Dibutyldecansäure, 2,2-Dimethylnonansäure, 2,2 Diethylnonansäure, 2,2-Dipropylnonansäure, 2,2-Dibutylnonansäure, 2,2-Dimethyloctansäure, 2,2-Diethyloctansäure, 2,2-Dipropyloctansäure, 2,2-Dibutyloctansäure, 2,2-Dimethylheptansäure, 2,2-Diethylheptansäure, 2,2-Dipropylheptansäure, 2,2-Dibutylheptansäure, 2,2-Dimethylhexansäure, 2,2-Diethylhexansäure, 2,2-Dipropylhexansäure, 2,2-Dibutylhexansäure, 2-Butyloctansäure, 2-Hexyldecansäure, 2-Propylpentan-säure, 1-Methyl-1-cyclohexancarbonsäure, 2,2-Dimethylbuttersäure, 2,2-Dimethylvaleriansäure, 3,5,5-Trimethylhexansäure, 2-Ethylhexansäure, Decansäure, Octansäure, Hexansäure und Önanthsäure bestehenden Gruppe ausgewählten Säure vorzusehen.

Gemäß einer weiteren bevorzugten Ausführungsform enthält das Zweikomponenten-Dentalmaterial als Katalysator b) wenigstens ein Salz von Diazabicyclo[5.4.0]undec-7-en, Diazabicyclo[4.3.0]non-5-en und/oder 1,1,3,3-Tetramethylguanidin mit 2-Alkylalkansäure, insbesondere 2-Alkylhexansäure, 2-Ethylhexansäure, 2,2-Dialkylalkansäure, 2,2-Dialkylhexansäure, 2,2-Dialkylnonansäure, 2,2-Dimethylhexansäure, 2,2-Diethylhexansäure, 2,2-Dimethylnonansäure, 2,2-Diethylnonansäure und/oder 2-Propylpentansäure.

In Weiterbildung des Erfindungsgedankens wird vorgeschlagen, dass die in den erfindungsgemäß einzusetzenden Katalysatorsalzen vorgesehenen Kationen und/oder Carbonsäureanionen Alkoxysilylgruppen aufweisen. Dadurch wird erreicht, dass das Katalysatorsalz nach Aushärten des Dentalmaterials in der Polyethermatrix eingebunden ist und aus dem dentalen Abformmaterial nicht mehr herausgelöst werden kann.

Erfindungsgemäß können die Zweikomponenten-Dentalmaterialien als Katalysator b) eines oder, in beliebiger Kombination miteinander, mehrere der zuvor genannten Salze enthalten. Vorzugsweise enthält das Zweikomponenten-Dentalmaterial nur eines der zuvor genannten Salze als Katalysator, wobei besonders bevorzugt neben dem einen oder mehreren erfindungsgemäß einzusetzenden Salzen keine weiteren Katalysatoren, insbesondere keine metallorganischen Metallsalze, tertiären Amine oder freie Säuren, eingesetzt werden.

Vorzugsweise wird in dem erfindungsgemäßen Zweikomponenten-Dentalmaterial ein Katalysatorsalz b) mit einem in Wasser (Ampuwa, pH 5,8) gemessenen pH-Wert zwischen 7 und 11 und besonders bevorzugt zwischen 7 und 9 eingesetzt.

Wie der Fachmann erkennt hängt die Menge an einzusetzendem Katalysatorsalz u.a. von der Löslichkeit des Salzes in der eingesetzten Polyethermatrix ab. Vorzugsweise beträgt die Menge an einzusetzendem Katalysatorsalz, bezogen auf die Gesamtmischung des Dentalmaterials, 0,001 bis 1 mmol/g, besonders bevorzugt 0,001 bis 0,5 mmol/g, ganz besonders bevorzugt 0,001 bis 0,1 mmol/g und höchst bevorzugt 0,005 bis 0,05 mmol/g. Selbstverständlich muss das eingesetzte Katalysatorsalz eine Mindestlöslichkeit in der eingesetzten Polyethermatrix aufweisen, um überhaupt katalytisch wirken zu können.

Um die Menge an einzusetzendem Katalysatorsalz möglichst gering zu halten, wird in Weiterbildung des Erfindungsgedankens vorgeschlagen, in dem Zweikomponenten-Dentalmaterial ein Katalysatorsalz mit einer hinreichend hohen Löslichkeit in dem Polyethermaterial, d.h. mit einer ausreichenden katalytischen Aktivität, einzusetzen, wobei die katalytische Aktivität wie im Zusammenhang mit der ersten besonderen Ausführungsform der vorliegenden Erfindung beschrieben ermittelt wird.

Gemäß einer dritten besonderen Ausführungsform der vorliegenden Erfindung enthält das Zweikomponenten-Dentalmaterial als Katalysator b) ein Salz, welches gebildet ist aus einer schwachen organischen Base mit einem in Wasser gemessenen pK_{BH+}-Wert zwischen -1 und 7 und wenigstens einer starken Säure mit einem in Wasser gemessenen pKs-Wert kleiner 2.

Gemäß einer ersten bevorzugten Unterausführungsform der dritten besonderen Ausführungsform der vorliegenden Erfindung ist der wenigstens eine Katalysator ein Salz, welches gebildet ist aus einer schwachen organischen Base mit einem in Wasser gemessenen pK_{BH+}-Wert zwischen -1 und 7 und wenigstens einer starken Säure mit einem in Wasser gemessenen pKs-Wert kleiner 2, wobei die Säure eine Struktur aufweist, welche nach Deprotonierung der Säure eine Mesomeriestabilisierung der negativen Ladung ermöglicht.

Das im erfindungsgemäßen Zweikomponenten-Dentalmaterial eingesetzte Katalysatorsalz ist vorzugsweise gebildet aus wenigstens einer Säure mit einem in Wasser gemessenen pKs-Wert von kleiner 1 und besonders bevorzugt von kleiner gleich 0,7 und/oder wenigstens einer Base mit einem in Wasser gemessenen pK_{BH+}-Wert zwischen 1 und 7, besonders bevorzugt zwischen 2 und 6 und ganz besonders bevorzugt zwischen 3 und 6.

Gemäß einer zweiten bevorzugten Unterausführungsform der dritten besonderen Ausführungsform der vorliegenden Erfindung ist das wenigstens eine Katalysatorsalz gebildet aus einer aus der aus p-Toluolsulfonsäure, Fluorsulfonsäure, Trifluormethansulfonsäure, Fluoroschwefelsäure, 4-Sulfophthalsäure, Trichloressigsäure, Trifluoressigsäure, Benzolsulfonsäure und Kombinationen hiervon bestehenden Gruppe ausgewählten Säure und/oder einer aus der aus Pyrrolderivaten, Dimethylanilin, Pyridin, 2,4,6-N,N-Pentamethylanilin, N,N-Dimethylanilin, Phenetedin, Acridin, Phenanthridin, Chinolin, Isochinolin, 2-Amino-4,6-dimethylpyrazin, 4,6-Dimethylpyridinamin, 3-Methylpyridin(3-picolin), 4-Phenylpyridin, 4-Vinylpyridin, Pyridazin, 2-Ethylpyridin, 2-Butylpyridin, 1,7-Phenanthrolin, 2-Aminopyrimidin, 2-Isopropylpyridin, 2-Vinylpyridin, 2-N,N-Dimethyl-aminopyridin, Chinazolin, 4-Chloropyridin, Phenazin, 4-Acetylpyridin, Methylnicotinat, 3-Benzoylpyridin, 2,2'-Bipyridin, 2-Phenylpyridin, 2-tert-Butylpyridin, Pyrimidin, 3-lodopyridin, 3-Fluoropyridin, 3-Chloropyridin, 3-Bromopyridin, Pyrazin, 7,8-Benzochinolin, 2-Chloropyridin, 4-Cyanopyridin und Kombinationen hiervon bestehenden Gruppe ausgewählten Base. Besonders bevorzugt wird als Katalysator ein Salz von p-Toluolsulfonsäure mit Pyridin oder ein Salz von p-Toluolsulfonsäure mit 2,4,6-N,N-Pentamethylanilin eingesetzt.

In Weiterbildung des Erfindungsgedankens wird vorgeschlagen, dass die in den erfindungsgemäß einzusetzenden Katalysatorsalzen vorgesehenen Kationen und/oder Säureanionen Alkoxysilylgruppen aufweisen. Dadurch wird erreicht, dass das Katalysatorsalz nach Aushärten des Dentalmaterials in der Polyethermatrix eingebunden ist und aus dem dentalen Abformmaterial nicht mehr herausgelöst werden kann.

Erfindungsgemäß können die Zweikomponenten-Dentalmaterialien als Katalysator b) eines oder, in beliebiger Kombination miteinander, mehrere der zuvor genannten Salze enthalten. Vorzugsweise enthält das Zweikomponenten-Dentalmaterial nur eines der zuvor genannten Salze als Katalysator, wobei besonders bevorzugt neben dem einen oder mehreren erfindungsgemäß einzusetzenden Salzen keine weiteren Katalysatoren, insbesondere keine metallorganischen Metallsalze, tertiären Amine oder freie Säuren, eingesetzt werden.

Vorzugsweise wird in dem erfindungsgemäßen Zweikomponenten-Dentalmaterial ein Katalysatorsalz b) mit einem in Wasser (Ampuwa, pH 5,8) gemessenen pH-Wert zwischen 1 und 7, besonders bevorzugt zwischen 2 und 6 und ganz besonders bevorzugt zwischen 2 und 5 eingesetzt.

Wie der Fachmann erkennt hängt die Menge an einzusetzendem Katalysatorsalz u.a. von der Löslichkeit des Salzes in der eingesetzten Polyethermatrix ab. Vorzugsweise beträgt die Menge an einzusetzendem Katalysatorsalz, bezogen auf die Gesamtmischung des Zweikomponenten-Dentalmaterials, 0,0005 bis 0,5 mmol/g, besonders bevorzugt 0,0005 bis 0,25 mmol/g und ganz besonders bevorzugt 0,0005 bis 0,05 mmol/g. Selbstverständlich muss das eingesetzte Katalysatorsalz eine Mindestlöslichkeit in der eingesetzten Polyethermatrix aufweisen, um überhaupt katalytisch wirken zu können.

Um die Menge an einzusetzendem Katalysatorsalz möglichst gering zu halten, wird in Weiterbildung des Erfindungsgedankens vorgeschlagen, in dem Zweikomponenten-Dentalmaterial ein Katalysatorsalz mit einer hinreichend hohen Löslichkeit in dem Polyethermaterial, d.h. mit einer ausreichenden katalytischen Aktivität, einzusetzen, wobei die katalytische Aktivität wie im Zusammenhang mit der ersten besonderen Ausführungsform der vorliegenden Erfindung beschrieben ermittelt wird.

Das Zweikomponenten-Dentalmaterial enthält in der Katalysatorkomponente B vorzugsweise Wasser c), wohingegen die Basiskomponente A möglichst absolut wasserfrei ist. Vorzugsweise enthält die Katalysatorkomponente B des erfindungsgemäßen Zweikomponenten-Dentalmaterials, bezogen auf die Gesamtmischung, 0,005 bis 3 mmol/g, besonders bevorzugt 0,01 bis 2 mmol/g und ganz besonders bevorzugt 0,02 bis 1 mmol/g an Wasser c).

Vorzugsweise enthält das erfindungsgemäße Zweikomponenten-Dentalmaterial wenigstens einen verstärkenden Füllstoff e₁) und/oder wenigstens einen nichtverstärkenden Füllstoff e₂). Dabei kann die

Basiskomponente A wenigstens einen der vorgenannten Füllstoffe enthalten, wobei vorzugsweise sowohl in der Basiskomponente A als auch in der Katalysatorkomponente B wenigstens ein verstärkender Füllstoff und/oder wenigstens ein nicht-verstärkender Füllstoff vorgesehen ist.

Als verstärkende Füllstoffe e₁) eignen sich insbesondere hochdisperse, aktive Füllstoffe mit einer BET-Oberfläche von wenigstens 50 m²/g und/oder einer Primärpartikelgröße von kleiner gleich 100 nm, besonders bevorzugt kleiner gleich 80 nm. Besonders geeignet sind solche mit einer Primärpartikelgröße im Nanometerbereich, welche als Aggregate und/oder Agglomerate vorliegen können. Bevorzugt ist der wenigstens eine verstärkende Füllstoffe e₁) eine Substanz ausgewählt aus der Gruppe, welche aus Aluminiumhydroxid, Zinkoxid, Titandioxid, Zirkoniumoxid, Siliciumdioxid sowie gefällter und/oder pyrogener Kieselsäure besteht. Selbstverständlich können die zuvor genannten Verbindungen einzeln oder in beliebiger Kombination miteinander eingesetzt werden, und zwar auch sowohl in hydrophiler als auch in hydrophobierter Form.

Ferner bevorzugt liegt der wenigstens eine verstärkende Füllstoff e₁) in Form von Nanopartikeln, als faser- oder blättchenförmiger Füllstoff, bspw. mineralischer, faserförmiger Füllstoff, oder als synthetischer, faserförmiger Füllstoff vor.

In Weiterbildung des Erfindungsgedankens wird vorgeschlagen, vorzugsweise in der Basiskomponente A, verstärkende Füllstoffe e₁) vorzusehen, die einen Wassergehalt von maximal 0,5 Gew.-%, besonders bevorzugt von maximal 0,3 Gew.-%, ganz besonders bevorzugt von maximal 0,15 Gew.-% aufweisen und höchst bevorzugt absolut bzw. im Wesentlichen wasserfrei sind, wobei der Wassergehalt über Karl-Fischer Titration bestimmt wird.

Gemäß einer besonderen Ausführungsform der vorliegenden Erfindung, weist der wenigstens eine verstärkende Füllstoff e₁) mit einer BET-Oberfläche von größer 50 m²/g in der Basiskomponente A einen pH-Wert von 5 bis 11, bevorzugt von 5 bis 9 und besonders bevorzugt von 5,5 bis 8,5, auf. Auf diese Weise wird eine Degradierung der alkoxysilylfunktionellen und/oder hydroxysilylfunktionellen Polyether während der Lagerung vermieden.

Im erfindungsgemäßen Zweikomponentensystem enthält vorzugsweise die Basiskomponente A, bezogen auf die Komponente A, 0 bis 50 Gew.-%, besonders bevorzugt 0,1 bis 40 Gew.-% und ganz besonders bevorzugt 0,1 bis 30 Gew.-%, und die Katalysatorkomponente B, bezogen auf die Komponente B, 0 bis 50 Gew.-%, besonders bevorzugt 0,1 bis 40 Gew.-% und ganz besonders bevorzugt 0,1 bis 30 Gew.-% wenigstens einen verstärkenden Füllstoff e₁).

Als nichtverstärkende Füllstoffe e₂) eignen sich prinzipiell dieselben Substanzen wie für die verstärkende Füllstoffe e₁), wobei die nichtverstärkenden jedoch zwingend eine BET-Oberfläche von weniger als 50 m²/g (Schriftenreihe Pigmente Degussa Kieselsäuren, Nummer 12, Seite 5 sowie Nummer 13, Seite 3) aufweisen. Bevorzugt ist der wenigstens eine nichtverstärkende Füllstoffe e₂) eine Substanz ausgewählt aus der Gruppe, welche aus Erdalkalimetalloxiden, Erdalkalimetallhydroxiden, Erdalkalimetallfluorid, Erdalkalimetallcarbonaten, Calciumapatit (Ca₅[(F, Cl, OH, ½CO₃) | (PO₄)₃], insbesondere Calciumhydroxylapatit (Ca₅[(OH) | (PO₄)₃]), Titandioxid, Zirkoniumoxid, Aluminiumhydroxid, Siliciumdioxid, gefällter Kieselsäure und Calciumcarbonat besteht. Selbstverständlich können die zuvor genannten Verbindungen einzeln oder in beliebiger Kombination miteinander eingesetzt werden, und zwar auch sowohl in hydrophiler als auch in hydrophobierter Form.

Vorzugsweise weisen die eingesetzten nichtverstärkenden Füllstoffe e₂) eine mittlere Korngröße von größer als 0,1 µm (Ullmann Encyclopädie der Technischen Chemie, Band 21, Seite 523) auf.

In Weiterbildung des Erfindungsgedankens wird beim erfindungsgemäßen Zweikomponentensystem vorgeschlagen, dass der wenigstens eine nichtverstärkende Füllstoff e₂) in der Basiskomponente A einen Wassergehalt von maximal 0,5 Gew.-%, besonders bevorzugt maximal 0,1 Gew.-%, ganz besonders bevorzugt maximal 0,05 Gew.-% aufweist und höchst bevorzugt absolut bzw. im Wesentlichen wasserfrei ist.

Gemäß einer besonderen Ausführungsform der vorliegenden Erfindung weist der wenigstens eine nichtverstärkende Füllstoff e₂) in der Basiskomponente A einen pH-Wert von 5 bis 11, bevorzugt von 5 bis 9 und besonders bevorzugt von 5,5 bis 8,5, auf, um eine Degradierung der alkoxysilylfunktionellen und/oder hydroxysilylfunktionellen Polyether während der Lagerung zu vermeiden.

Vorzugsweise enthält die Basiskomponente A des erfindungsgemäßen Dentalmaterials, bezogen auf die Komponente A, 0 bis 80 Gew.-%, besonders bevorzugt 0,05 bis 75 Gew.-% und ganz besonders bevorzugt 0,1 bis 70 Gew.-%, und die Katalysatorkomponente B, bezogen auf die Komponente B, 0 bis 80 Gew.-%, besonders bevorzugt 0,05 bis 75 Gew.-% und ganz besonders bevorzugt 0,1 bis 70 Gew.-% wenigstens eines nichtverstärkenden Füllstoffs e₂).

In Weiterbildung des Erfindungsgedankens wird vorgeschlagen, dass beim erfindungsgemäßen Zweikomponentenmaterial die in der Katalysatorkomponente B enthaltenen verstärkenden und/oder nichtverstärkenden Füllstoffe einen pH-Wert zwischen 6,0 und 11,0 und ganz besonders bevorzugt solche mit einem pH-Wert zwischen 7,0 und 10,0 aufweisen.

Insgesamt beträgt der Gesamtgehalt an Füllstoffen sowohl beim erfindungsgemäßen Zweikomponentensystem, bezogen auf die Gesamtmischung, 0 bis 80 Gew.-%, bevorzugt 0,01 bis 80 Gew.-%, besonders bevorzugt 0,1 bis 75 Gew.-% und ganz besonders bevorzugt 0,2 bis 70 Gew.-%.

In Weiterbildung des Erfindungsgedankens wird vorgeschlagen, dem erfindungsgemäßen Zweikomponenten-Dentalmaterial ein oder mehrere der folgenden Zusatz- bzw. Hilfsstoffe zuzusetzen:
f) Thixotropierungsmittel,
g) Wasserfänger,
h) Pastenbildner,
i) Tensid,
j) Wirkstoff,
k) Weichmacher,
l) optische Abtastung ermöglichende Substanz,
m) Geschmacks- und/oder Geruchsstoff,
n) Diagnostik ermöglichende Substanz,
o) Fluoridisierungsmittel,
p) Bleichsubstanz,
q) Desensibilisierungsmittel,
r) Haftverbundvermittler,
s) Farbstoff,
t) Indikator,
u) Stabilisator (Antioxidanz, Radikalfänger).

Dem erfindungsgemäßen Zweikomponenten-Dentalmaterial können optional Thixotropierungsmittel f) zugesetzt werden, wobei sich zu diesem Zweck insbesondere hochmolekulare Polyether, wie Polyethylenglykol, Polyethylenglykol-Polypropylenglykol-Copolymere, Poly-Tetrahydrofuran, Kohlenwasserstoffwachse, Amidwachse, Triglyceride, Kieselsäuren und Silicate als besonders geeignet erwiesen haben.

Gemäß einer besonderen Ausführungsform der vorliegenden Erfindung weisen die Dentalmaterialien, vorzugsweise in der Basiskomponente A, wenigstens einen Wasserfänger g) auf, welcher besonders bevorzugt aus der Gruppe, welche aus Alkoxysilanen, Titanaten, wie Tetraisopropyltitanat, Zirkonaten, wie Tetrapropylzirkonat, Zeolithen, Aluminiumsulfat, wasserfreiem Calciumsulfat (bspw. Drierite^{®}), Blaugel und/oder Oxazolidinen besteht, ausgewählt ist.

In Weiterbildung des Erfindungsgedankens wird vorgeschlagen, als Wasserfänger g) ein oder mehrere funktionelle Alkoxysilane einzusetzen, da durch solche Verbindungen zusätzlich die Vernetzungsgeschwindigkeit, die Struktur und die Eigenschaften des resultierenden Elastomers eingestellt werden können. Bevorzugt ist das wenigstens eine funktionelles Alkoxysilan eine Verbindung der allgemeinen Formel V

R⁸₄₋ₓ-Si-R⁹ₓ

mit R⁸=H, Alkyl, Alkenyl, -(CH₂)ₙ-Z, wobei n=1 bis 6,
R⁹=Alkoxy,
Z=NH₂, NHR, NR₂, wobei R=Alkyl, Aminoalkyl, -C(O)OCH₃, sowie x=1, 2, 3 oder 4,
wobei besonders bevorzugt R⁸= Alkenyl, insbesondere Vinyl, oder -(CH₂)ₙ-Z mit Z=NHR und n=1 oder 3, insbesondere n=1, und/oder x=3 und/oder R= -C(O)OCH₃.

Besonders bevorzugt ist das wenigstens eine funktionelle Alkoxysilan g) Vinyltrimethoxysilan, N-Trimethoxysilylmethyl-O-methylcarbamat und/oder eine Verbindung der folgenden Formel: 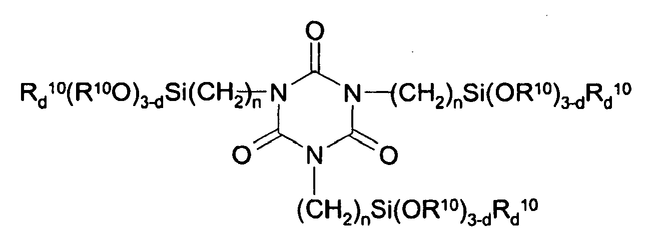 worin n = 1 bis 6, bevorzugt n = 1 oder 3, besonders bevorzugt n = 1, d = 0 oder 1, und
R¹⁰ = ein linearer oder verzweigter C₁-C₃₀-Alkylrest, in dem die Wasserstoffatome teilweise durch Halogenatome, OH-, NH₂-, NO₂- oder auch weitere C₁-C₆-Alkylreste substituiert sein können, sind.

Die zuvor genannten Verbindungen sind reaktive Silane, die als Wasserfänger zur Beseitigung von in der Komponente A der Dentalzusammensetzung noch vorhandenen Wasserspuren fungieren.

Des weiteren enthalten die erfindungsgemäßen Zweikomponenten-Dentalmaterialien vorzugsweise in der Katalysatorkomponente B, wenigstens einen Pastenbildner h), da dieser die Einstellung einer pastenartigen Konsistenz, bspw. dünn-, mittel- oder zähfließend, sowie eine homogene Vermischung des Salzes und der festen verstärkenden und nichtverstärkenden Füllstoffen ermöglicht. Vorzugsweise wird als wenigstens ein Pastenbildner h) eine Verbindung ausgewählt aus der Gruppe, welche aus Polyethern, Polyvinylpyrrolidonen, Polyurethanen, Polyestern, Wachsen, Vaseline, Paraffinölen, Siliconölen, mehrwertigen Alkoholen, Propylenglycolen, Polypropylenglycolen, Ethylenglycolen, Polyethylenglycolen, Copolymerisaten aus N-Vinylpyrrolidon und Vinylacetat, Carboxymethyl-, Methyl-, Hydroxyethyl-, Hydroxypropyl-Cellulose, Polysacchariden, Glycerin und Poly(meth)acrylsäuren besteht, eingesetzt. Selbstverständlich können die erfindungsgemäßen Zweikomponenten-Dentalmaterialien auch eine beliebige Kombination zweier oder mehrerer der zuvor genannten Verbindungen enthalten.

Besonders bevorzugt sind hydrophile Pastenbildner, in denen sich die erfindungsgemäß eingesetzte Katalysatorbase mit Wasser homogen mischen lässt. Die Mischbarkeit kann durch Zusatz von Tensiden noch verbessert werden. Besonders bevorzugte Vertreter sind Polyether, Polyurethane, Polyester, mehrwertige Alkohole, insbesondere Propylenglycole, Polypropylenglycole, Ethylenglycole, Polyethylenglycole, Butylenglycole, Polybutylenglycole und Glycerin, sowie Mischungen und Copolymere hiervon.

Bei den ggf. als Tensid, Emulgator und/oder Stabilisator eingesetzten Verbindungen i) handelt es sich vorzugsweise um anionische Tenside, besonders bevorzugt Alkylsulfate, Alkylbenzolsulfonate oder Alkylbenzolphosphate, kationische Tenside, besonders bevorzugt Tetraalkylammoniumhalogenide, nichtionische Tenside, besonders bevorzugt Alkyl- und Alkylphenyl-Polyalkylalkylenoxide, Fettsäurealkoxylate, Fettalkoholalkyloxylate sowie deren Alkylether und Alkylester, Fettsäurealkylolamide, Saccharosefettsäureester, Trialkylaminoxide, Silicontenside (z.B. Silwet L77, Tegopren 5878, Masil SF19) oder Fluortenside, oder um amphotere Tenside, besonders bevorzugt sulfatierte oder oxyethylierte Kondensationsprodukte aus Alkenylphenolen und Formaldehyd, Ethylenoxid-propylenoxid-Blockpolymerisate oder modifizierte Polysiloxane. Mit Vorteil können auch in den alkoxysilylfunktionellen und/oder hydroxysilylfunktionellen Polyether a) einpolymerisierbare Tenside, wie sie in der US 4,160,778 offenbart sind, eingesetzt werden. Zusätzlich oder alternativ dazu können auch

Derivate der zuvor genannten Tenside eingesetzt werden, bspw. solche, die über funktionelle Gruppen, wie -OH, -CH=CH₂, -OCO-(CH₃)C=CH₂ sowie Alkoxysilylgruppen, verfügen. Zudem können, wenn auch weniger bevorzugt, andere, dem Fachmann bekannte Tenside eingesetzt werden.

Bevorzugt wird als eingesetzten Verbindungen i) ein Silicontensid eingesetzt, da sich im Rahmen der vorliegenden Erfindung gezeigt hat, dass mit diesen Verbindungen überraschenderweise in der Polyethermatrix sehr niedrige, mit der Methode "liegender Tropfen" bestimmte, Kontaktwinkel erzielt werden können.

Diese Mischungen zeichnen sich durch eine ausgezeichnete Benetzbarkeit und ein hervorragendes Anfließverhalten an feuchte Zahn- und Gewebssubstanz aus. Trotz dieser guten hydrophilen Eigenschaften quillt das Material nicht bei Kontakt mit wässrigen Medien, wie Wasser, Speichel, Blut, Desinfektionsbad oder wässrigem Gipsbrei. Die gute initiale Benetzbarkeit der Mischungen ist wichtig für die detailgetreue Abformung des Abformmaterials in dem Patientenmund während der Verarbeitung und dem ersten Kontakt mit feuchter Mund-/Zahnsubstanz und drückt sich durch einen niedrigen Kontaktwinkel von kleiner 50°, insbesondere kleiner gleich 45°, gemessen mit einem Kontaktwinkelmessgerät der Firma Krüss bei 20 °C mit der Messmethode "liegender Tropfen", aus. Zudem zeichnet sich auch das ausgehärtete Abformmaterial zum Zeitpunkt des Ausgießens mit Gips (direkt oder 2 Stunden nach dem Aushärten) durch einen Kontaktwinkel von kleiner 60°, insbesondere kleiner gleich 55°, aus.

Zudem können die erfindungsgemäßen Zweikomponenten-Dentalmaterialien eine oder mehrere Wirkstoffe j) enthalten, welche in Abhängigkeit von deren chemischen Funktionalität in der Basiskomponente A oder der Katalysatorkomponente B enthalten sein können. Zu den erfindungsgemäß einzusetzenden Wirkstoffen zählen insbesondere Adstringentien, wie Epinephrine, antibakteriell und/oder antifugal wirkende Substanzen, wie Hexitidine (bspw. 5-Amino-1, 3-bis(2-ethylhexyl)-5-methylhexahydropyrimidin), Triclosane (bspw. 2,4,4'-Trichloro-2-hydroxydiphenylether) und Chlorhexidin: 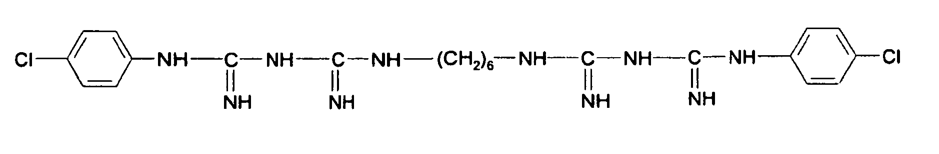 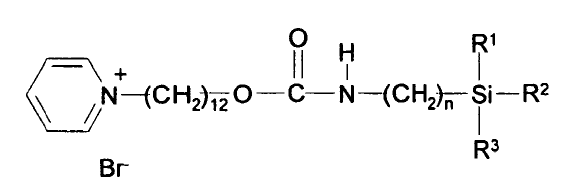 worin R¹, R² und R³ unabhängig voneinander Alkoxy-, Alkyl-, Aryl-, Aralkyl-, Alkylarylgruppen oder Wasserstoff, bevorzugt Butoxy-, Propoxy-, Ethoxy-, Methoxy-, Hexyl-, Pentyl-, Butyl-, Propyl-, Ethyl- oder Methylgruppen, besonders bevorzugt Ethoxy-, Methoxy-, Ethyl- oder Methylgruppen, sind mit der Maßgabe, dass mindestens einer der vorgenannten Reste, vorzugsweise zwei oder alle drei Reste, Alkoxygruppen sind.

Als Weichmacher k) kommen insbesondere nicht reaktive Polyether, Polydialkylsiloxane, Siliconöle, Polyester, Polyurethane, Phthalate, Mono-, Di-, Tri- oder höherwertige Ester, insbesondere Acetyltributylcitrat, Mesamoll^{®} (Bayer) und Triglyceride in Betracht, welche bei Formulierung als Zweikomponentensystem je nach deren chemischen Natur der Komponente A und/oder der Komponente B zugesetzt werden.

Als die optische Lesbarkeit/Abtastung mittels Scanner bzw. intraoraler Kamera (Cerec^{®}, Fa. Sirona) ermöglichenden Verbindungen I) können alle dem Fachmann zu diesem Zweck bekannte Substanzen, insbesondere Metallpulver, Metallpigmente, Metallicpigmente, Zinkoxid, Zirkonoxid und Titandioxid, eingesetzt werden, welche bei Formulierung als Zweikomponentensystem je nach deren chemischen Natur der Komponente A und/oder der Komponente B zugesetzt werden.

Zudem können die erfindungsgemäßen Zweikomponenten-Dentalmaterialien in einer der beiden oder in beiden Komponenten übliche Geschmacks- und/oder Geruchsstoffe m) und/oder für die Diagnostik nützliche Zusätze n) enthalten, wie sie bspw. in der EP 1 339 373, PCT/EP00/05418, DE 35 02 594 und DE 100 61 195 beschrieben sind.

Als Fluoridierungshilfsstoffe o) haben sich insbesondere Natriumfluorid, Kaliumfluorid, Ammoniumfluorid, Fluorphosphate und Aminfluoride, wie N'-Octadecyl-bimethylendiamin-N, N, N'-bis(2-ethanol)-dihydrofluorid (wie in ZM 93, Nummer 15, Seite 32 ff. beschrieben), als geeignet erwiesen, welche bei Formulierung als Zweikomponentensystem ebenfalls in Abhängigkeit von deren chemischen Natur der Komponente A und/oder der Komponente B zugesetzt sein können.

Zudem kann das erfindungsgemäße Zweikomponenten-Dentalmaterial in der Komponente A und/oder der Komponente B, als Bleichsubstanz p) eine oder mehrere verschiedene Peroxide enthalten, welche vorzugsweise aus der Gruppe, welche aus Alkalimetall- und Erdalkalimetallperoxiden, Wasserstoffperoxid sowie Carbamidperoxid besteht, ausgewählt sind.

Beispiele für geeignete Desensibilisierungsmittel q) sind Kaliumsalze, wie Kaliumnitrat, Nelkenöl und Eugenol.

Als Haftverbundvermittler r), bspw. zur Ausbildung eines Haftverbundes zwischen dem Abformmaterial und einem Abformlöffel aus Edelstahl und/oder Kunststoff, eignen sich insbesondere Alkoxysilane, Epoxysilane, Aminosilane und Methacrylatsilane.

Beispiele für geeignete Farbstoffe s) sind Farbstoffpigmente in Form von Al-, Ca-, Ba-Oxiden/verlackter Farbstoff, welche wie die zuvor beschriebenen Hilfsstoffe, sofern nicht anders angegeben, bei Formulierung als Zweikomponentensystem in Abhängigkeit von deren chemischen Natur der Komponente A und/oder der Komponente B zugesetzt sein können.

Des weiteren können dem erfindungsgemäßen Zweikomponenten-Dentalmaterial in der Komponente A und/oder der Komponente B Farbstoffindikatoren t) zugesetzt sein, welche ihre Farbe in Abhängigkeit von dem pH-Wert, bspw. aufgrund von pH-Wert-Änderungen beim Vermischen der Komponenten A und B, oder beim Kontakt mit Wasser ändern.

Als Stabilisatoren und/oder Antioxidantien u) können den erfindungsgemäßen Zweikomponenten-Dentalmaterialien insbesondere aus der aus polymerem Trimethyl-dihydrochinolin, Diphenylderivaten, Phenothiazin, Phenyl-αnaphthylamin, 4, 4'-Methylen-bis-2,6-di-tert.-butylphenol, Butylhydroxytoluol, Butylhydroxyanisol (BHA) und Methoxyphenol (Hydroxyanisol) bestehenden Gruppe ausgewählte Verbindungen eingesetzt werden. Beispiele für derartige Verbindungen sind die von der Firma Ciba-Geigy kommerziell erhältlichen Produkte Irganox 1010, 1076, 1035, MD 1024, Irgafos 168, 38, Irgacor 252 LD/252FC, 1405, 1930, 153, Tinuvin 328, P, 384, 900, 928, 327, 1130, 400, 292, 144, 123, 622 sowie Chimassorb 119.

Vorzugsweise wird das erfindungsgemäße Zweikomponenten-Dentalmaterial in geeigneten Primärverpackungen, wie Tuben, Dosen, und besonders bevorzugt in Kartuschen und Schlauchbeuteln, wie sie bspw. in der EP 0 723 807 A2, EP-A-0 541 972, PCT/EP/980193, EP-A-0 492 412, EP-A-0 492 413 und EP 0 956 908 A1 beschrieben sind, gelagert und auf die spätere Verwendung zugeschnitten proportioniert ist.

Ein weiterer Gegenstand der vorliegenden Erfindung sind Mischungen, welche durch Vermischen der Komponenten A und B des zuvor beschriebenen erfindungsgemäßen Zweikomponenten-Dentalmaterials erhältlich sind. Vorzugsweise wird die Basiskomponente A mit der Katalysatorkomponente B in einem Verhältnis von 1:1 bis 20:1, besonders bevorzugt von 1:1 bis 10:1 und ganz besonders bevorzugt von 1:1, 2:1, 4:1 oder 5:1, vermischt.

Im Folgenden wird die Erfindung anhand von den Erfindungsgedanken demonstrierenden, diesen aber nicht einschränkenden Beispielen erläutert.

### Beispiel 1 (erfindungsgemäß)

*(Herstellung eines formstabilen Vulkanisats*/*Formkörpers eines Dentalabformmaterials auf Basis von alkoxysilylfunktionellen und*/*oder hydroxysilylfunktionellen Polyethern durch Zusatz von Ethylendiamintetraessigsäure als saure Verbindung d) in der Basiskomponente A)*

### Herstellung der Katalysatorkomponente B

2,80 Teile eines Salzes aus 1,9-Diazabicyclo[5.4.0]undec-7-en und 2-Ethylhexansäure wurden in 5 Teilen entmineralisiertem Wasser (Ampuwa, pH 5,8) gelöst. Die Salzlösung wurde in einem Vakuummischbecher mit 36 Teilen Polypropylenglycol mit einer Molmasse von 4.000 g/mol, 51 Teilen Aluminiumhydroxid mit einer mittleren Korngröße von 13 µm und einer BET-Oberfläche von 1 m²/g und 5 Teilen einer Kieselsäure mit einer BET-Oberfläche von 130 m²/g für 5 Minuten gemischt. Danach wurde weitere 30 Minuten unter Vakuum homogen gemischt.

Es wurde ein mittelfließendes Material (ISO 4823) erhalten, das die Katalysatorkomponente B des erfindungsgemäßen Abformmaterials auf Basis von Alkoxysilylpolyethern darstellt. Das Material wurde in Schlauchbeutel (Verbundfolie PE/AI/PE) abgefüllt und aufbewahrt.

### Herstellung der Basiskomponente A

In einem Vakuummischer wurden unter trockener Argon-Schutzgasatmosphäre 39 Teile eines Polypropylenglycols, das endständig über Urethangruppen und Methylenspacer mit Dimethoxymethylsilylgruppen funktionalisiert war, wobei das funktionalisierte Polypropylenglycol eine Viskosität bei 20°C von 10.000 mPa·s aufwies, mit 51 Teilen eines getrockneten mit Trimethylsilylgruppen oberflächenmodifizierten Cristobalitfüllstoffs mit einer mittleren Korngröße von 7 µm, zwei Teilen einer getrockneten, hochdispersen, pyrogen hergestellten, hydrophobierten Kieselsäure mit einer BET-Oberfläche von 170 m²/g, 0,4 Teilen Vinyltrimethoxysilan und 0,4 Teilen N-Trimethoxysilylmethyl-O-methylcarbamat, zwei Teilen Silicontensid und 0,8 Teilen Ethylendiamintetraessigsäure für 5 Minuten gemischt. Danach wurde weitere 30 Minuten unter Vakuum homogen gemischt.

Es wurde ein mittelfließendes Material (ISO 4823) erhalten, das die Basiskomponente A des erfindungsgemäßen Abformmaterials auf Basis von Alkoxysilylpolyethern darstellt. Das Material wurde in Schlauchbeutel (Verbundfolie PE/AI/PE) abgefüllt und aufbewahrt.

### Mischung der Katalysatorkomponente B und der Basiskomponente A

Jeweils 1 Teil der zuvor beschriebenen Katalysatorkomponente B und 5 Teile der gemäß obiger Vorschrift hergestellten Basiskomponente A wurden mit Hilfe eines elektrischen Austraggeräts aus Schlauchbeuteln (Plug & Press System, Kettenbach GmbH & Co. KG) über einen dynamischen Mischer (Kettenbach GmbH & Co. KG) homogen gemischt und ausgehärtet.

Das ausgehärtete Vulkanisat wies folgende anwendungstechnischen Eigenschaften auf:

| | |
|---|---|
| Shore A-Härte: | unmittelbar nach Abbindeende: 52 nach 24 h: 61 |
| Rückstellung nach Verformung: | 98,6 % |
| Konsistenz der Katalysator-Komponente B: | 38 mm |
| Konsistenz der Basis-Komponente A: | 36 mm |
| Konsistenz der Mischung: | 35 mm |
| lineare Maßänderung: | - 0,42 % |
| Kontaktwinkel 45 Sek. nach Mischbeginn: | 40° |
| Verarbeitungszeit: | 2'00" |
| Abbindezeit: | 6'00" |
| Deformation I: | 0,00 mm |

Zur Prüfung der Formstabilität im Sinne der Erfindung wurden quaderförmige Prüfkörper des ausgehärteten Dentalabformmaterials aus Komponente A und B mit einer Abmessung von 40 x 12,5 x 5 mm und einer Shore A-Härte von 52 (unmittelbar nach Abbindeende) auf einem Objektträger einseitig befestigt, so dass eine Hälfte, also 20 x 12,5 x 5 mm, ungestützt über dem Objektträger hinausragt und der Schwerkraft seines Eigengewichtes ausgesetzt ist.

Die unterstützte bzw. die ungestützte Fläche des Prüfkörpers wurde so gewählt, dass keine spontane Deformation alleine durch die Schwerkraft auftritt. Die hier angegebene Unterstützung auf der Hälfte, also 20 x 12,5 x 5 mm, der Prüfkörperfläche hat sich für Prüfkörper mit einer Shore A-Härte von 50 bis 70 bewährt. Weichere Prüfkörper mit einer Shore A-Härte von 30 - 50 sollten mit einer größeren Unterstützungsfläche von 70 %, also 28 x 12,5 x 5 mm, und entsprechend kleineren ungestützten Fläche von 30 %, also 12 x 12,5 x 5 mm, aufgebracht werden. In bestimmten Zeitabständen wurde die vertikale Auslenkung I (Deformation) des Prüfkörpers zu dessen ursprünglicher Ausgangsposition gemessen.

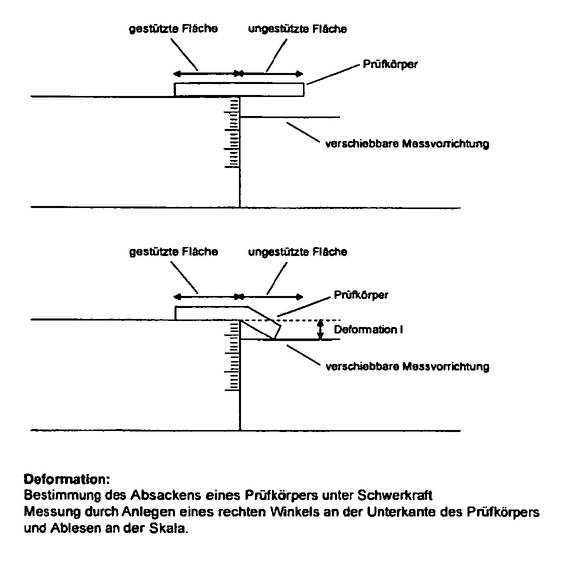

Wie die vorgenannten technischen Daten des ausgehärteten Vulkanisats zeigen, erfüllen die erfindungsgemäßen Materialien alle Anforderungen für ein funktionsfähiges Dentalabformmaterial. Insbesondere die Hydrophilie, die durch Kontaktwinkelmessungen bestimmt wird, zeigt hervorragende Werte. Ferner zeichnet sich das in diesem Beispiel erhaltene Material dadurch aus, dass die erhaltenen Vulkanisate/Formkörper auch nach Lagerung für 2 Wochen bei 60°C bzw. für 3 Monate bei Raumtemperatur formstabil sind.

Überraschenderweise wird die Lagerstabilität der Basiskomponente durch den Zusatz von Ethylendiamintetraessigsäure nicht beeinträchtigt, d. h. es treten weder eine vorzeitige Vernetzung noch eine Viskositätserhöhung während der Lagerzeit auf.

Die in Beispiel 1 verwendete Ethylendiamintetraessigsäure hat eine Wasserlöslichkeit von 0,5 g/l bei 20°C und liegt damit im erfindungsgemäßen Bereich. Zudem weist Ethylendiamintetraessigsäure in Wasser folgende pKₛ-Werte auf: pKₛ₁=2,0, pKₛ₂=2,67 und pKₛ₃=6,16. Diese drei pKₛ-Werte liegen innerhalb des erfindungsgemäß bevorzugten pKₛ-Wert-Bereiches von 1 bis 6,5 Zudem liegt die Polyetherlöslichkeit mit kleiner gleich 2 g/l bzw. kleiner gleich 0,5 g/l in dem erfindungsgemäß bevorzugten Bereich.

Die anwendungstechnischen Eigenschaften des in Beispiel 1 erhaltenen Materials sind in der Tabelle 1 zusammengefasst. Zudem ist in der Tabelle 2 der Einfluss der erfindungsgemäß eingesetzten sauren Verbindung auf die über Rückstellung nach der Verformung gemessene Abbindezeit im Vergleich zu der von Verkaufsprodukten nach dem Stand der Technik wiedergegeben. Aus der Tabelle 2 ist ersichtlich, dass der Zusatz einer sauren Verbindung überraschenderweise keinen negativen Einfluss auf die Abbindezeit ausübt.

### Beispiel 2 (erfindungsgemäß)

*(Herstellung eines formstabilen Vulkanisats*/*Formkörpers eines Dentalabformmatenals auf Basis von alkoxysilylfunktionellen undloder hydroxysilylfunktionellen Polyethern durch Zusatz von Ethylendiamintetraessigsäure als saure Verbindung d) in der Basiskomponente A)*

Es wurde analog zu Beispiel 1 vorgegangen, wobei die Menge an Ethylendiamintetraessigsäure auf 0,56 Teile reduziert wurde.

Die erhaltenen Vulkanisate/Formkörper waren nach Lagerung für zwei Wochen bei 60°C bzw. für 3 Monate bei Raumtemperatur formstabil.

Überraschenderweise wird die Lagerstabilität der Basiskomponente A durch den Zusatz von Ethylendiamintetraessigsäure nicht beeinträchtigt, d. h. es treten weder eine vorzeitige Vernetzung noch eine Viskositätserhöhung während der Lagerzeit auf.

Die anwendungstechnischen Eigenschaften des in Beispiel 2 erhaltenen Materials sind in der Tabelle 1 zusammengefasst. Zudem ist in der Tabelle 2 der Einfluss der erfindungsgemäß eingesetzten sauren Verbindung auf die über Rückstellung nach der Verformung gemessene Abbindezeit im Vergleich zu der von Verkaufsprodukten nach dem Stand der Technik wiedergegeben. Aus der Tabelle 2 ist ersichtlich, dass der Zusatz einer sauren Verbindung überraschenderweise keinen negativen Einfluss auf die Abbindezeit ausübt.

### Beispiel 3 (erfindungsgemäß)

*(Herstellung eines formstabilen Vulkanisats*/*Formkörpers eines Dentalabformmaterials auf Basis von alkoxysilylfunktionellen und*/*oder hydroxysilylfunktionellen Polyethern durch Zusatz von Barbitursäure als saure Verbindung d) in der Basiskomponente A)*

### Herstellung der Katalysatorkomponente B

Als Katalysatorkomponente B wurde die Katalysatorkomponente aus Beispiel 1 verwendet.

### Herstellung der Basiskomponente A

Als Basiskomponente A wurde die Basiskomponente aus Beispiel 1 verwendet, wobei anstelle von Ethylendiamintetraessigsäure 0,28 Teile Barbitursäure eingesetzt wurden.

### Mischung der Katalysatorkomponente B und der Basiskomponente A

Jeweils 1 Teil der zuvor beschriebenen Katalysatorkomponente B und 5 Teile der gemäß obiger Vorschrift hergestellten Basiskomponente A wurden mit Hilfe eines elektrischen Austraggeräts aus Schlauchbeuteln (Plug & Press System, Kettenbach GmbH & Co. KG) über einen dynamischen Mischer (Kettenbach GmbH & Co. KG) homogen gemischt und ausgehärtet.

Zur Prüfung der Formstabilität wurde analog zu Beispiel 1 vorgegangen.

Es zeigte sich, dass die erhaltenen Vulkanisate/Formkörper auch nach einer Lagerung für eine Woche bei 60°C bzw. für einen Monat bei Raumtemperatur formstabil waren.

Überraschenderweise wird die Lagerstabilität der Basiskomponente A durch den Zusatz von Barbitursäure nicht beeinträchtigt, d. h. es treten weder eine vorzeitige Vernetzung noch eine Viskositätserhöhung während der Lagerzeit auf.

Die in Beispiel 3 verwendete Barbitursäure hat eine Wasserlöslichkeit von 142 g/l bei 20°C und liegt damit innerhalb des erfindungsgemäßen Bereiches. Zudem weist diese in Wasser einen innerhalb des erfindungsgemäß bevorzugten pKₛ-Wert-Bereiches von 1 bis 6,5 liegenden pKₛ-Wert von 4,01 auf. Weiterhin liegt die Polyetherlöslichkeit mit kleiner gleich 2 g/l ebenfalls im erfindungsgemäß bevorzugten Bereich.

Die anwendungstechnischen Eigenschaften des in Beispiel 3 erhaltenen Materials sind in der Tabelle 1 zusammengefasst.

### Beispiel 4 (erfindungsgemäß)

*(Herstellung eines formstabilen Vulkanisats*/*Formkörpers eines Dentalabformmaterials auf Basis von alkoxysilylfunktionellen und*/*oder hydroxysilylfunktionellen Polyethern durch Zusatz von Bernsteinsäureanhydrid als saure Verbindung d) in der Basiskomponente A)*

### Herstellung der Katalysatorkomponente B

Als Katalysatorkomponente B wurde die Katalysatorkomponente aus Beispiel 1 verwendet.

### Herstellung der Basiskomponente A

Als Basiskomponente A wurde die Basiskomponente aus Beispiel 1 verwendet, wobei anstelle von Ethylendiamintetraessigsäure 0,11 Teile Bernsteinsäureanhydrid eingesetzt wurden

### Mischung der Katalysatorkomponente B und der Basiskomponente A

Jeweils 1 Teil der zuvor beschriebenen Katalysatorkomponente B und 5 Teile der gemäß obiger Vorschrift hergestellten Basiskomponente A wurden mit Hilfe eines elektrischen Austraggeräts aus Schlauchbeuteln (Plug & Press System, Kettenbach GmbH & Co. KG) über einen dynamischen Mischer (Kettenbach GmbH & Co. KG) homogen gemischt und ausgehärtet.

Zur Prüfung der Formstabilität wurde analog Beispiel 1 vorgegangen.

Es zeigte sich, dass de erhaltenen Vulkanisate/Formkörper auch nach einer Lagerung für eine Woche bei 60°C bzw. für einen Monate bei Raumtemperatur formstabil waren.

Überraschenderweise wird die Lagerstabilität der Basiskomponente A durch den Zusatz von Bernsteinsäureanhydrid nicht beeinträchtigt, d. h. es treten weder eine vorzeitige Vernetzung noch eine Viskositätserhöhung während der Lagerzeit auf.

Das in Beispiel 4 verwendete Bernsteinsäureanhydrid wird nach dem Mischvorgang durch das Wasser aus der Katalysatorkomponente B in Bernsteinsäure gespalten, die mit 4,19 einen innerhalb des erfindungsgemäß bevorzugten pKs-Wert-Bereiches von 1 bis 6,5 liegenden pKₛ-Wert aufweist.

Die anwendungstechnischen Eigenschaften des in Beispiel 4 erhaltenen Materials sind in der Tabelle 1 zusammengefasst.

### Vergleichsbeispiel 1

*(Herstellung eines Vulkanisats*/*Formkörpers eines Dentalabformmaterials auf Basis von alkoxysilylfunktionellen und*/*oder hydroxysilylfunktionellen Polyethern ohne Zusatz einer sauren Verbindung d) in der Basiskomponente A)*

### Herstellung der Katalysatorkomponente B

Als Katalysatorkomponente B wurde die Katalysatorkomponente aus Beispiel 1 verwendet.

### Herstellung der Basiskomponente A

Als Basiskomponente A wurde die Basiskomponente aus Beispiel 1 verwendet, wobei auf den Zusatz von formstabilisierender Ethylendiamindiamintetraessigsäure verzichtet wurde.

### Mischung der Katalysatorkomponente B und der Basiskomponente A

Jeweils 1 Teil der zuvor beschriebenen Katalysatorkomponente B und 5 Teile der gemäß obiger Vorschrift hergestellten Basiskomponente A wurden mit Hilfe eines elektrischen Austraggeräts aus Schlauchbeuteln (Plug & Press System, Kettenbach GmbH & Co. KG) über einen dynamischen Mischer (Kettenbach GmbH & Co. KG) homogen gemischt und ausgehärtet.

Die technischen Daten des in Vergleichsbeispiel 1 erhaltenen ausgehärteten Abfommaterials entsprachen hinsichtlich der Shore A-Härte unmittelbar nach Abbindeende (52) denen des in Beispiel 1 erhaltenen Materials.

Die Prüfung der Formstabilität erfolgte ebenso wie bei Beispiel 1.

Die nach dem Aushärten erhaltenen Vulkanisate/Formkörper deformierten (zerflossen sogar teilweise) bereits nach 10 Tagen Lagerung bei 60°C bzw. nach 4 Wochen Lagerung bei Raumtemperatur um mehr als 0,5 mm. Ein entsprechender Zahnabdruck wird nach dieser Lagerung unbrauchbar, da verformte oder zerflossene Abdrücke das Zahnmodell verfälschen und würde zu nicht brauchbarem Zahnersatz führen. Schon aus diesem Grund ist das in Vergleichsbeispiel 1 erhaltene Material als Dentalabformmaterial ungeeignet.

Die anwendungstechnischen Eigenschaften des in Vergleichsbeispiel 1 erhaltenen Materials sind in der Tabelle 1 zusammengefasst.

### Vergleichsbeispiel 2

*(Versuche zur Herstellung von Vulkanisaten*/*Formkörpern eines Dentatabformaterials auf Basis von a*/*koxysi*/*ylfunktionellen und*/*oder hydroxysilylfunktionellen Polyethern mit Zusatz von p-Toluolsulfonsäure zur Basiskomponente A)*

### Herstellung der Katalysatorkomponente B

Als Katalysatorkomponente B wurde die Katalysatorkomponente aus Beispiel 1 verwendet.

### Herstellung der Basiskomponente A

Als Basiskomponente A wurde die Basiskomponente aus Beispiel 1 verwendet, wobei die 0,8 Teile Ethylendiamintetraessigsäure durch 0,52 Teile p-Toluolsulfonsäure ersetzt werden.

Bereits beim Einmischen der p-Toluolsulfonsäure begann die Mischung zu vernetzen. Wenige Minuten nach Zugabe der Säure war das Material vollständig ausgehärtet.

Dieses Beispiel zeigt eindrucksvoll, dass nicht jede saure Verbindung zur Erzielung von formstabilen Vulkanisaten/Formkörpern geeignet ist. Die in diesem Beispiel verwendete p-Toluolsulfonsäure weist mit ca. 750 g/l eine außerhalb des erfindungsgemäßen Bereichs liegende Wasserlöslichkeit (20°C) auf. Zudem liegt der pKₛ-Wert in Wasser mit 0,7 sehr niedrig und außerhalb des erfindungsgemäß bevorzugten Bereiches von 1,0 bis 6,5. Darüber hinaus ist die Polyetherlöslichkeit von p-Toluolsulfonsäure vergleichsweise hoch. Insbesondere aufgrund der hohen Wasserlöslichkeit erfolgt bei diesem Material bereits in der Herstellphase eine vorzeitigen Vernetzung des Alkoxysilyl- und/oder Hydroxysilylpolyethers. Die Herstellung eines lagerfähigen Dentalabformmaterials ist daher nicht möglich ebenso wenig wie die Erstellung von Prüfkörpern zur Untersuchung der Formstabilität.

Die anwendungstechnischen Eigenschaften des in Vergleichsbeispiel 2 erhaltenen Materials sind in der Tabelle 1 zusammengefasst.

### Vergleichsbeispiel 3

*(Versuche zur Herstellung von Vulkanisaten*/*Formkörpern eines Dentatabformaterials auf Basis von a*/*koxysilylfunktionellen und*/*oder hydroxysilylfunktionellen Polyethern mit Zusatz von Phosphorsäure zur Basiskomponente A)*

### Herstellung der Katalysatorkomponente B

Als Katalysatorkomponente B wurde die Katalysatorkomponente aus Vergleichsbeispiel 1 verwendet.

### Herstellung der Basiskomponente A

Als Basiskomponente A wurde die Basiskomponente aus dem erfindungsgemäßen Beispiel 1 verwendet, wobei die 0,8 Teile Ethylendiamintetraessigsäure durch 0,27 Teile 30 %-ige Phosphorsäure ersetzt wurden.

Beim Einmischen der Phosphorsäure begann die Mischung zu vernetzen. Bereits wenige Minuten nach Zugabe der Säure war das Material vollständig ausgehärtet. Eine Herstellung eines lagerfähigen Dentalabformmaterials war mit diesem Material ebenso wenig möglich wie die Erstellung von Prüfkörpern zur Untersuchung der Formstabilität.

Auch dieses Vergleichsbeispiel zeigt, dass nicht jedes saure Mittel zur Erzielung von formstabilen Vulkanisaten/Formkörpern geeignet ist. Die in diesem Beispiel verwendete Phosphorsäure weist mit 750 g/l eine ebenfalls weit außerhalb des erfindungsgemäß vorgesehenen Bereichs liegende Wasserlöslichkeit auf.

Die anwendungstechnischen Eigenschaften des in Vergleichsbeispiel 1 erhaltenen Materials sind in der Tabelle 1 zusammengefasst.

**Tabelle 1 Formstabiliät und Einfluss auf Lagerstabilität der Basis-Komponente A von sauren Verbindungen in basen- bzw. salzkatalysiert kondensationsvernetzenden Zweikomponenten-Dentalabformmaterialien auf Basis von alkoxysilylfunktionellen Polyethern**

| **Beispiel/ Vergleichs- beispiel** | **saure Verbindung** | **Konzentration [%]** | **Lagerstabilität¹⁾ der Basis-Komponente A** | **Abbindeverhalten nach Mischen der Komponenten A + B** | **Formstabilität des ausgehärteten Vulkanisats** | **Deformation I⁴⁾ 10 Tage 60°C** | **elastische Rück- stellung nach Verformung** | | | **Shore A Härte** | | | **Eignung** |
|---|---|---|---|---|---|---|---|---|---|---|---|---|---|
| | | | | | | | **AM⁵⁾** | **1W⁶⁾ 60°C** | **2W⁶⁾ 60°C** | **AM⁵⁾** | **1W⁶⁾ 60°C** | **2W⁶⁾ 60°C** | |
| Beispiel 1 | Ethylendiamin-tetraessigsäure CAS-Nr. 60-00-4 | 0,8 | lagerstabil: | - keine Beschleunigung keine | keine Deformation formstabil | 0,00 mm | 98,6 | 99,9 | 99,9 | 68 | 70 | 70 | + + + |
| | | | - keine Viskositätserhöhung | | | | | | | | | | |
| | | | - keine Aushärtung | - Inhibierung | | | | | | | | | |
| Beispiel 2 | Ethylendiamin- tetraessigsäure | 0,56 | lagerstabil: | - keine Beschleunigung | keine Deformation formstabil | 0,00 mm | 98,7 | 99,9 | 99,9 | 68 | 70 | 70 | +++ |
| | | | - keine Viskositätserhöhung | | | | | | | | | | |
| | | | - keine Aushärtung | - keine Inhibierung | | | | | | | | | |
| Beispiel 3 | Barbitursäure | 0,28 | lagerstabil: | - keine Beschleunigung | sehr geringe Deformation formstabil | 0,05 mm | 98,5 | 99,9 | 99,9 | 68 | 70 | 70 | + + |
| | | | - geringe Viskositäts-erhöhung | | | | | | | | | | |
| | | | - keine Aushärtung | - geringe Inhibierung | | | | | | | | | |
| Beispiel 4 | Bernsteinsäure- anhydrid | 0,11 | lagerstabil: | - keine Beschleunigung | geringe Deformation formstabil | 0,1 mm | 98,2 | 99,8 | 99,8 | 68 | 70 | 70 | + |
| | | | - geringe Viskositäts-erhöhung | | | | | | | | | | |
| | | | - keine Aushärtung | - geringe Inhibierung | | | | | | | | | |
| Vergleichs- beispiel 1 | ohne Zusatz | 0 | lagerstabil: | - keine Beschleunigung | nicht formstabil | 0,58 mm | 99,3 | 99,9 | -³⁾ | 69 | 66 | 61 | - - - |
| | | | - keine Viskositätserhöhung | | | | | | | | | | |
| | | | - keine Aushärtung | - keine Inhibierung | | | | | | | | | |
| Vergleichs- beispiel 2 | p-Toluolsulfon- säure | 0,52 | vernetzt direkt nach Zugabe | nicht möglich²⁾ | nicht möglich²⁾ | | | - | | | | | - - - |
| Vergleichs- beispiel 3 | Phosphorsäure | 0,27 | vernetzt direkt nach Zugabe | nicht möglich²⁾ | nicht möglich²⁾ | | | - | | | | | - - - |

| | | | | | | | | | | | | | |
|---|---|---|---|---|---|---|---|---|---|---|---|---|---|
| - - - ungeeignet +++ sehr gut geeignet ¹⁾Lagerung bei Raumtemperatur und bei 60°C (1 Woche, 2 Wochen, 3 Wochen, 4 Wochen) ²⁾ Material vernetzt unmittelbar nach Zugabe des sauren Mittels in der Basis-Komponente A, deshalb Mischen mit Komponente B nicht möglich ³⁾ nicht messbar, da Prüfkörper zerflossen ⁵⁾ Ausgangsmessung ⁶⁾ 1 bis 2 Wochen Lagerzeit | | | | | | | | | | | | | |

**Tabelle 2: Abbindeendebestimmung über Rückstellung nach der Verformung angelehnt an ISO 4823 (Fassung von 1992)**

| | Aziridinopolyether 1 | Aziridnopolyether 2 | Alkoxysilylpolyether 1 | A-Silicon | Alkoxysilylpolyether Beispiel 2 | Alkoxysilylpolyether Beispiel 1 | Alkoxysilylpolyether Vergleichsbeispiel 1 |
|---|---|---|---|---|---|---|---|
| | Base LOT 166056 Cat LOT 166145 | Base LOT 174886 Cat LOT 174697 | Base LOT 200047 Cat LOT 195039 | Base LOT 0409000261 Cat LOT 0409000261 | erfindungsgemäß | erfindungsgemäß | nicht erfindungsgemäß |
| | | | | | 0,56% Ethylen- diamintetra- essigsäure | 0,8% Ethylen- diamintetra- essigsäure | ohne Ethylen- diamintetra- essigsäure |
| Angabe Packungsbeilage | Abbindeende: 6'00" | Abbindeende: 6'00" | Abbindeende: mind.5'15" | Abbindeende: mind. 5'15" | ----- | ----- | ----- |
| R.n.d.V. nach | | | | | | | |
| | | | | | | | |
| 6'00" | 98,15% | 98,70% | 98,30% | 98,40% | 98,70% | 98,60% | 98,90% |
| 5'00" | 97,50% | 97,40% | 98,00% | 98,00% | 98,20% | 98,00% | 98,10% |
| 4'00" | 96,40% | 96,30% | 97,10% | 97,90% | 95,90% | 96,80% | 94,80% |
| | | | | | | | |
| 98,00 % ^{1), 2), 3)} erreicht bei: | 5'45" | 5'30" | 5'00" | 5'00" | 5'00" | 5'15" | 5'00" |

| | | | | | | | |
|---|---|---|---|---|---|---|---|
| Aziridinopolyether 1: Impregum penta (ESPE) Aziridinopolyether 2: Impregum penta soft (ESPE) Alkoxysilylpolyether 1: P2 Magnum 360 Monophase (Heraeus Kulzer) A-Silicon: Aquasil ultra Monophase (Dentsply) ¹⁾ errechnet durch Interpolation ²⁾ der Wert 98,00 % wurde als vernünftiger Standardwert gewählt, bei dem eine qualitativ hochwertige Präzisionsabformung erstellt werden kann ³⁾ das Abbindeende wird auf 15 Sekunden auf- oder abgerundet; die Bestimmung erfolgte durch Messung der Rückstellung nach der Verformung gemäß ISO 4823 (Fassung von 1992) | | | | | | | |

## Patentansprüche

1. Kondensationsvernetzendes Zweikomponenten-Dentalmaterial mit einer Komponente A enthaltend
a) wenigstens einen alkoxysilylfunktionellen und/oder hydroxysilylfunktionellen Polyether
und einer Komponente B enthaltend
b) wenigstens einen der aus Basen, Salzen und beliebigen Kombinationen hiervon bestehenden Gruppe ausgewählten Katalysator,
**dadurch gekennzeichnet, dass** die Komponente A des weiteren wenigstens eine saure Verbindung d) enthält, welche eine Wasserlöslichkeit bei 20°C von nicht mehr als 150 g/l aufweist.

2. Dentalmaterial nach Anspruch 1, **dadurch gekennzeichnet, dass** Komponente B Wasser c) enthält.

3. Dentalmaterial nach einem der Ansprüche 1 oder 2, **dadurch gekennzeichnet, dass** die wenigstens eine saure Verbindung d) aus der aus organischen Säuren, anorganischen Säuren, anorganischen Säureanhydriden, organischen Säureanhydriden und beliebigen Kombinationen hiervon bestehenden Gruppe ausgewählt ist.

4. Dentalmaterial nach einem der vorhergehenden Ansprüche, **dadurch gekennzeichnet, dass** die wenigstens eine saure Verbindung d) eine Wasserlöslichkeit bei 20°C von nicht mehr als 50 g/l aufweist.

5. Dentalmaterial nach Anspruch 4, **dadurch gekennzeichnet, dass** die wenigstens eine saure Verbindung d) eine Wasserlöslichkeit bei 20°C von nicht mehr als 0,5 g/l aufweist.

6. Dentalmaterial nach einem der vorhergehenden Ansprüche, **dadurch gekennzeichnet, dass** die wenigstens eine saure Verbindung d) eine Polyetherlöslichkeit bei 20°C von nicht mehr als 2 g/l aufweist, wobei unter Polyetherlöslichkeit die bei 20°C gemessene Löslichkeit der sauren Verbindung d) in einem Polyether der nachfolgenden Formel zu verstehen ist

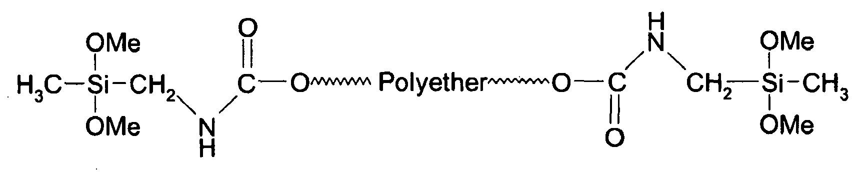

7. Dentalmaterial nach Anspruch 6, **dadurch gekennzeichnet, dass** die wenigstens eine saure Verbindung d) eine Polyetherlöslichkeit bei 20°C von nicht mehr als 0,5 g/l aufweist, auf, wobei unter Polyetherlöslichkeit die bei 20°C gemessene Löslichkeit der sauren Verbindung d) in einem_Polyether der nachfolgenden Formel zu verstehen ist:

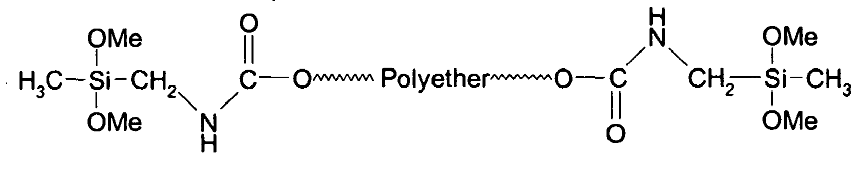

8. Dentalmaterial nach einem der vorhergehenden Ansprüche, **dadurch gekennzeichnet, dass** die wenigstens eine saure Verbindung d) einen in Wasser bei 20°C gemessenen pKₛ-Wert zwischen 1,0 und 6,5 aufweist.

9. Dentalmaterial nach Anspruch 8, **dadurch gekennzeichnet, dass** die wenigstens eine saure Verbindung d) einen in Wasser bei 20°C gemessenen pKₛ-Wert zwischen 1,5 und 4,3 aufweist.

10. Dentalmaterial nach einem der vorhergehenden Ansprüche, **dadurch gekennzeichnet, dass** die wenigstens eine saure Verbindung d) aus der aus Barbitursäure, Bernsteinsäureanhydrid, Ethylendiamintetraessigsäure, Derivaten von Ethylendiamintetraessigsäure, Mono-, Di- und Tri-Alkalisalzen von Ethylendiamintetraessigsäure, Mono-, Di- und Tri-Erdalkalisalzen von Ethylendiamintetraessigsäure und beliebigen Kombinationen hiervon bestehenden Gruppe ausgewählt ist.

11. Dentalmaterial nach einem der vorhergehenden Ansprüche, **dadurch gekennzeichnet, dass** das Dentalmaterial, bezogen auf die Gesamtmischung, 0,001 bis 10 Gew.-% der wenigstens einen sauren Verbindung d) enthält.

12. Dentalmaterial nach Anspruch 11, **dadurch gekennzeichnet, dass** das Dentalmaterial, bezogen auf die Gesamtmischung, 0,2 bis 1,5 Gew.-% der wenigstens einen sauren Verbindung d) enthält.

13. Dentalmaterial nach einem der vorhergehenden Ansprüche, **dadurch gekennzeichnet, dass** dieses als Katalysator b) eine organische Base mit einem in Acetonitril gemessenen pK_{BH+}-Wert von mindestens 20 enthält.

14. Dentalmaterial nach Anspruch 13, **dadurch gekennzeichnet, dass** die wenigstens eine Katalysatorbase b) aus der aus 1,1,3,3-Tetramethylguanidin (TMG), 1,8- Diazabicyclo[5.4.0]undec-7-en (DBU), 1,5-Diazabicyclo[4.3.0]non-5-en (DBN), tert-Butylimino-tris(dimethylamino)-phosphoran, tert-Butylimino-tri(pyrrolidino)phosphoran, tert-Octylimino-tris(dimethylamino)phosphoran, 2-tert-Butylimino-2-diethylamino-1,3-dimethyl-perhydro- 1,3,2- diazaphosphorin, 2-tert-Butylimino-2-diethylamino-1,3-dimethyl-perhydro- 1,3,2-diazaphosphorin auf Polystyrol, 1-tert-Butyl-2,2,4,4,4-pentakis(diethylamino)-2Λ5, 4Λ5-catenadi(phosphazen), 1-Ethyl-2,2,4,4,4-pentakis(diethylamino)-2Λ5, 4Λ5-catenadi(phosphazen), 1-tert-Butyl-4,4,4-tris(dimethylamino)-2,2-bis[tris-(dimethylamino)-phosphor-anylidenamino]-2Λ⁵, 4Λ⁵-catenadi(phosphazen), 1-tert-Octyl-4,4,4-tris(dimethylamino)-2,2-bis[tris-(dimethylamino)-phosphor-anylidenamino]-2Λ⁵,4Λ⁵-catenadi(phosphazen), 2,8,9-Triisobutyl-2,5,8,9-tetraaza-1-phosphabicyclo[3.3.3]undecan, 2,8,9-Tri-isopropyl-2,5,8,9-tetraaza-1-phosphabicyclo[3.3.3]undecan, 2,8,9-Trimethyl-2,5,8,9-tetraaza-1-phosphabicyclo[3.3.3]undecan, 2-tert-Butyl-1,1,3,3-tetramethylguanidin, 1,8-Bis(tetramethylguanidino)naphthalen (TMGN),1,5,7-Triazabicyclo(4.4.0)dec-5-en, 7-Methyl-1,5,7-triazabicyclo(4.4.0)dec-5-en, 1,5-Diazabicyclo[4.3.0)non-5-en und 3,3,6,9,9-Pentamethyl-2,10-diazabicyclo-(4.4.0)dec-1-en bestehenden Gruppe ausgewählt ist.

15. Dentalmaterial nach Anspruch 13 oder 14, **dadurch gekennzeichnet, dass** dieses, bezogen auf die Gesamtmischung des Dentalmaterials, 0,001 bis 1 mmol/g einer Katalysatorbase b) enthält.

16. Dentalmaterial nach einem der Ansprüche 1 bis 12, **dadurch gekennzeichnet, dass** dieses als Katalysator b) wenigstens ein Salz enthält, welches gebildet ist aus wenigstens einem aus der aus
- Komplexen von Alkalimetall- oder Ammonium-Kationen mit Kronenethern und/oder Kryptanden,
- Tetraalkyl-, Tetraaryl-, Trialkylaryl-, Dialkyldiaryl-, Monoalkyltriarylammonium-Kationen, Tetraalkyl-, Tetraaryl-, Trialkyl-aryl-, Dialkyldiaryl-, Monoalkyltriarylphosphonium-Kationen, Tetraalkyl-, Tetraaryl-, Trialkylaryl-, Dialkyldiaryl-, Monoalkyltriarylarsonium-Kationen, Tetraalkyl-, Tetraaryl-, Trialkylaryl-, Dialkyldiaryl-, Monoalkyltriarylstibonium-Kationen,
- durch Protonierung einer Base mit einem in Acetonitril gemessenen pK_{BH+}-Wert von mindestens 20 gebildeten Kationen
und Kombinationen hiervon bestehenden Gruppe ausgewählten Kation und wenigstens einem Anion einer gesättigten und/oder ungesättigten (cyclo)aliphatischen Carbonsäure, wobei die Carbonsäure eine verzweigte Carbonsäure mit einer Länge der an der Carboxylgruppe vorgesehenen (Cyclo)Alkylkette von wenigstens 2 Kohlenstoffatomen oder eine unverzweigte Carbonsäure mit einer Länge der an der Carboxylgruppe vorgesehenen (Cyclo)Alkylkette von wenigstens 4 Kohlenstoffatomen ist.

17. Dentalmaterial nach Anspruch 16, **dadurch gekennzeichnet, dass** Anion des wenigstens einen Katalysatorsalzes b) ein Anion einer verzweigten Carbonsäure mit einer Länge der an der Carboxylgruppe vorgesehenen (Cyclo)Alkylkette von wenigstens 3 Kohlenstoffatomen oder einer unverzweigten Carbonsäure mit einer Länge der an der Carboxylgruppe vorgesehenen (Cyclo)Alkylkette von wenigstens 5 Kohlenstoffatomen ist.

18. Dentalmaterial nach Anspruch 17, **dadurch gekennzeichnet, dass** Anion des wenigstens einen Katalysatorsalzes b) ein Anion von cycloaliphatischen Monocarbonsäuren ist.

19. Dentalmaterial nach einem der Ansprüche 1 bis 12, **dadurch gekennzeichnet, dass** dieses als Katalysator b) wenigstens ein Salz enthält, welches gebildet ist aus einer schwachen organischen Base mit einem in Wasser gemessenen pK_{BH+}-Wert zwischen -1 und 7 und wenigstens einer starken Säure mit einem in Wasser gemessenen pKs-Wert kleiner 2.

20. Dentalmaterial nach Anspruch 19, **dadurch gekennzeichnet, dass** der wenigstens eine Katalysator b) ein Salz ist, welches gebildet ist aus einer schwachen organischen Base mit einem in Wasser gemessenen pK_{BH+}-Wert zwischen -1 und 7 und wenigstens einer starken Säure mit einem in Wasser gemessenen pKs-Wert kleiner 2, wobei die Säure eine Struktur aufweist, welche nach Deprotonierung der Säure eine Mesomeriestabilisierung der negativen Ladung ermöglicht.

21. Dentalmaterial nach Anspruch 19 oder 20, **dadurch gekennzeichnet, dass** das wenigstens eine eingesetzte Katalysatorsalz b) gebildet ist aus wenigstens einer Base mit einem in Wasser gemessenen pK_{BH+}-Wert zwischen 1 und 7 und/oder wenigstens einer Säure mit einem in Wasser gemessenen pKs-Wert von kleiner 1.

22. Dentalmaterial nach Anspruch 21, **dadurch gekennzeichnet, dass** das wenigstens eine eingesetzte Katalysatorsalz b) gebildet ist aus wenigstens einer Base mit einem in Wasser gemessenen pK_{BH+}-Wert zwischen 3 und 6 und/oder wenigstens einer Säure mit einem in Wasser gemessenen pKs-Wert von kleiner gleich 0,7.

23. Dentalmaterial nach einem der Ansprüche 19 bis 22, **dadurch gekennzeichnet, dass** der wenigstens eine Katalysator b) ein Salz ist gebildet aus einer aus der aus p-Toluolsulfonsäure, Fluorsulfonsäure, Trifluormethansulfonsäure, Fluoroschwefelsäure, 4-Sulfophthalsäure, Trichloressigsäure, Trifluoressigsäure, Benzolsulfonsäure und Kombinationen hiervon bestehenden Gruppe ausgewählten Säure und/oder einer aus der aus Pyrrolderivaten, Dimethylanilin, Pyridin, 2,4,6-N,N-Pentamethylanilin, N,N-Dimethylanilin, Phenetedin, Acridin, Phenanthridin, Chinolin, Isochinolin, 2-Amino-4,6-dimethylpyrazin, 4,6-Dimethylpyridinamin, 3-Methylpyridin(3-picolin), 4-Phenyl-pyridin, 4-Vinylpyridin, Pyridazin, 2-Ethylpyridin, 2-Butylpyridin, 1,7-Phenanthrolin, 2-Aminopyrimidin, 2-Isopropylpyridin, 2-Vinylpyridin, 2-N,N-Dimethylaminopyridin, Chinazolin, 4-Chloropyridin, Phenazin, 4-Acetylpyridin, Methylnicotinat, 3-Benzoylpyridin, 2,2'-Bipyridin, 2-Phenylpyridin, 2-tert-Butylpyridin, Pyrimidin, 3-lodopyridin, 3-Fluoropyridin, 3-Chloropyridin, 3-Bromopyridin, Pyrazin, 7,8-Benzochinolin, 2-Chloropyridin, 4-Cyanopyridin und Kombinationen hiervon bestehenden Gruppe ausgewählten Base.

24. Mischung erhältlich durch Vermischen der Komponenten A und B des Zweikomponenten-Dentalmaterials nach einem der Ansprüche 1 bis 23, **dadurch gekennzeichnet, dass** die Basiskomponente A mit der Katalysatorkomponente B in einem Verhältnis von 1:1 bis 20:1 vermischt wird.

25. Verwendung eines Dentalmaterials nach einem der Ansprüche 1 bis 23 zur Herstellung von Dentalabformmaterial.

## Claims

1. A condensation-cured two-component dental material having a component A containing:
a) at least one alkoxysilyl-functional and/or hydroxysilyl-functional polyether;
and a component B containing:
b) at least one catalyst selected from the group formed by bases, salts and any combination thereof;
**characterized in that** the component A further contains at least one acid d) which has a solubility in water of not more than 150 g/l at 20°C.

2. The dental material according to claim 1, **characterized in that** component B contains water c).

3. The dental material according to claim 1 or claim 2, **characterized in that** the at least one acid d) is selected from the group formed by organic acids, inorganic acids, inorganic acid anhydrides, organic acid anhydrides and any combination thereof.

4. The dental material according to one of the preceding claims, **characterized in that** the at least one acid d) has a solubility in water of not more than 50 g/l at 20°C.

5. The dental material according to claim 4, **characterized in that** the at least one acid d) has a solubility in water of not more than 0.5 g/l at 20°C.

6. The dental material according to one of the preceding claims, **characterized in that** the at least one acid d) has a solubility in polyether of not more than 2 g/l at 20°C, wherein the term "solubility in polyether" means the solubility, measured at 20°C, of the acid d) in a polyether with the following formula:

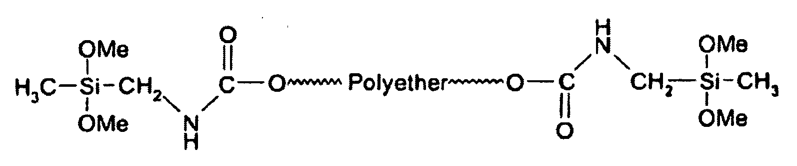

7. The dental material according to claim 6, **characterized in that** the at least one acid d) has a solubility in polyether of not more than 0.5 g/l at 20°C, wherein the term "solubility in polyether" means the solubility, measured at 20°C, of the acid d) in a polyether with the following formula:

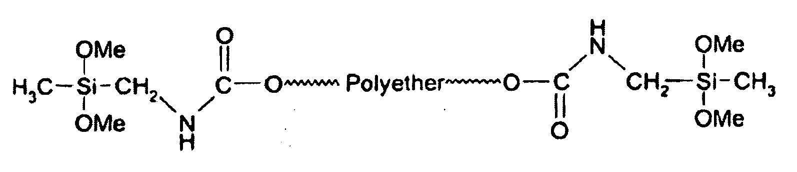

8. The dental material according to one of the preceding claims, **characterized in that** the at least one acid d) has a pKₐ value in the range 1.0 to 6.5, measured in water at 20°C.

9. The dental material according to claim 8, **characterized in that** the at least one acid d) has a pKₐ value in the range 1.5 to 4.3, measured in water at 20°C.

10. The dental material according to one of the preceding claims, **characterized in that** the at least one acid d) is selected from the group formed by barbituric acid, succinic acid anhydride, ethylenediaminetetraacetic acid, derivatives of ethylenediaminetetraacetic acid, mono-, di- and tri-alkali salts of ethylenediaminetetraacetic acid, mono-, di- and tri-alkaline-earth salts of ethylenediaminetetraacetic acid and any combinations thereof.

11. The dental material according to one of the preceding claims, **characterized in that** the dental material contains 0.001% to 10% by weight of the at least one acid d) with respect to the total mixture.

12. The dental material according to claim 11, **characterized in that** the dental material contains 0.2% to 1.5% by weight of the at least one acid d) with respect to the total mixture.

13. The dental material according to one of the preceding claims, **characterized in that** it contains, as catalyst b), an organic base with a pK_{BH+} value, measured in acetonitrile, of at least 20.

14. The dental material according to claim 13, **characterized in that** the at least one catalyst base b) is selected from the group formed by 1,1,3,3-tetramethylguanidine (TMG), 1,8-diazabicyclo[5.4.0]undec-7-ene (DBU), 1,5-diazabicyclo[4.3.0]non-5-ene (DBN), tert-butylimino-tris(dimethylamino)-phosphorane, tert-butylimino-tri(pyrrolidino)phosphorane, tert-octylimino-tris(dimethylamino)phosphorane, 2-tert-butylimino-2-diethylamino-1,3-dimethyl-perhydro-1,3,2-diazaphosphorine, 2-tert-butylimino-2-diethylamino-1,3-dimethylperhydro-1,3,2-diazaphosphorin on polystyrene, 1-tert-butyl-2,2,4,4,4-pentakis(diethylamino)-2A5,4A5-catenadi(phosphazene), 1-ethyl-2,2,4,4,4-pentakis(diethylamino)-2Λ5,4Λ5-catenadi(phosphazene), 1-tert-butyl-4,4,4-tris(dimethylamino)-2,2-bis[tris-(dimethylamino)-phosphoranylidenamino]-2Λ⁵,4Λ⁵-catenadi(phosphazene),1-tert-oxtyl-4,4,4-tris(dimethylamino)-2,2-bis[tris-(dimethylamino)-phosphoranylidenamino]-2Λ⁵,4Λ⁵-catenadi(phosphazene), 2,8,9-triisobutyl-2,5,8,9-tetra-aza-1-phosphabicyclo[3.3.3]undecane, 2,8,9-tri-isopropyl-2,5,8,9- tetra-aza-1-phosphabicyclo[3.3.3]undecane, 2,8,9-trimethyl-2,5,8,9- tetra-aza-1-phosphabicyclo[3.3.3]undecane, 2-tert-butyl-1,1,3,3-tetramethylguanidine, 1,8-bis(tetramethylguanidino)naphthalene (TMGN), 1,5,7-triazabicyclo(4.4.0)dec-5-ene, 7-methyl-1,5,7-triazabicyclo(4.4.0)dec-5-ene, 1,5-diazabicyclo[4.3.0]non-5-ene and 3,3,6,9,9-pentamethyl-2,10-diazabicyclo(4.4.0)dec-1-ene.

15. The dental material according to claim 13 or claim 14, **characterized in that** it contains 0.001 to 1 mmol/g of a catalyst base b) with respect to the total dental material mixture.

16. The dental material according to one of claims 1 to 12, **characterized in that** it contains at least one salt as catalyst b), said salt being formed by at least one cation selected from the group formed by:
- complexes of alkali metal or ammonium cations with crown ethers and/or cryptands;
- te-traalkyl-, tetraaryl-, trialkylaryl-, dialkyldiaryl-, monoalkyltriaryl-ammonium cations, tetraalkyl-, tetraaryl-, trialkylaryl-, dialkyldiaryl-, monoalkyltriaryl-phosphonium cations, tetraalkyl-, tetraaryl-, trialkylaryl-, dialkyldiaryl-, monoalkyltriaryl-arsonium cations, tetraalkyl-, tetraaryl-, trialkylaryl-, dialkyldiaryl-, monoalkyltriaryl-stibonium cations;
- cations formed by protonation of a base with a pK_{BH+} value of at least 20, measured in acetonitrile;
and combinations thereof, and at least one anion of a saturated and/or unsaturated (cyclo)aliphatic carbonic acid, wherein the carbonic acid is a branched carbonic acid with the (cyclo)alkyl chain provided on the carboxyl group having a length of at least 2 carbon atoms or a straight chain carbonic acid with the (cyclo)alkyl chain provided on the carboxyl group having a length of at least 4 carbon atoms.

17. The dental material according to claim 16, **characterized in that** the anion of the at least one catalyst salt b) is an anion of a branched carbonic acid with the (cyclo)alkyl chain provided on the carboxyl group having a length of at least 3 carbon atoms or of a straight chain carbonic acid with the (cyclo)alkyl chain provided on the carboxyl group having a length of at least 5 carbon atoms.

18. The dental material according to claim 17, **characterized in that** the anion of the at least one catalyst salt b) is an anion of cycloaliphatic monocarbonic acids.

19. The dental material according to one of claims 1 to 12, **characterized in that** it contains at least one salt as the catalyst b) which is formed from a weak organic base with a pK_{BH+} value in the range -1 to 7 measured in water and at least one strong acid with a pKₐ value of less than 2, measured in water.

20. The dental material according to claim 19, **characterized in that** the at least one catalyst b) is a salt which is formed from a weak organic base with a pK_{BH+} value in the range -1 to 7 measured in water and at least one strong acid with a pKₐ value of less than 2, measured in water, wherein the acid has a structure which, following deprotonation, permits mesomeric stabilization of the negative charge.

21. The dental material according to claim 19 or claim 20, **characterized in that** the at least one catalyst salt b) which is employed is formed from at least one base with a pK_{BH+} value in the range 1 to 7 measured in water and/or at least one acid with a pKₐ value of less than 1, measured in water.

22. The dental material according to claim 21, **characterized in that** the at least one catalyst salt b) which is employed is formed from at least one base with a pK_{BH+} value in the range 3 to 6 measured in water and/or at least one acid with a pKₐ value of 0.7 or less, measured in water.

23. The dental material according to one of claims 19 to 22, **characterized in that** the at least one catalyst b) is a salt formed from an acid selected from the group formed by p-toluenesulphonic acid, fluorosulphonic acid, trifluoromethanesulphonic acid, fluorosulphuric acid, 4-sulphophthalic acid, trichloroacetic acid, trifluoroacetic acid, benzenesulphonic acid and combinations thereof and/or from a base selected the group formed by pyrrole derivatives, dimethylaniline, pyridine, 2,4,6-N,N-pentamethylaniline, N,N-dimethylaniline, phenetidine, acridine, phenanthridine, quinoline, isoquinoline, 2-amino-4,6-dimethylpyrazine, 4,6-dimethylpyridinamine, 3-methylpyridine (3-picoline), 4-phenylpyridine, 4-vinylpyridine, pyridazine, 2-ethylpyridine, 2-butylpyridine, 1,7-phenanthroline, 2-aminopyrimidine, 2-isopropylpyridine, 2-vinylpyridine, 2-N,N-dimethylaminopyridine, quinazoline, 4-chloropyridine, phenazine, 4-acetylpyridine, methylnicotinate, 3-benzoylpyridine, 2,2'-bipyridine, 2-phenylpyridine, 2-tert-butylpyridine, pyrimidine, 3-iodopyridine, 3-fluoropyridine, 3-chloropyridine, 3-bromopyridine, pyrazine, 7,8-benzoquinoline, 2-chloropyridine, 4-cyanopyridine and combinations thereof.

24. A mixture which can be obtained by mixing components A and B of the two-component dental material according to one of claims 1 to 23, **characterized in that** the base component A is mixed with the catalyst component B in a ratio of 1:1 to 20:1.

25. Use of a dental material according to one of claims 1 to 23 for the manufacture of dental impression materials.

## Revendications

1. Matériau dentaire à deux composants subissant une réticulation par condensation, avec un composant A contenant
a) au moins un polyéther pourvu de fonctions alcoxysilyle et/ou de fonctions hydroxysilyle
et un composant B contenant
b) au moins un catalyseur choisi dans le groupe constitué de bases, de sels et d'une quelconque de leurs combinaisons,
**caractérisé en ce que** le composant A contient, en outre, au moins un composé acide d) dont la solubilité dans l'eau à 20 °C est inférieure ou égale à 150 g/l.

2. Matériau dentaire selon la revendication 1, **caractérisé en ce que** le composant B contient de l'eau c).

3. Matériau dentaire selon l'une des revendications 1 ou 2, **caractérisé en ce que** l'au moins un composé acide d) est choisi dans le groupe constitué des acides organiques, des acides inorganiques, des anhydrides d'acides inorganiques, des anhydrides d'acides organiques et d'une quelconque de leurs combinaisons.

4. Matériau dentaire selon l'une des revendications précédentes, **caractérisé en ce que** l'au moins un composé acide d) présente, à 20 °C, une solubilité dans l'eau inférieure ou égale à 50 g/l.

5. Matériau dentaire selon la revendication 4, **caractérisé en ce que** l'au moins un composé acide d) présente, à 20 °C, une solubilité dans l'eau inférieure ou égale à 0,5 g/l.

6. Matériau dentaire selon l'une des revendications précédentes, **caractérisé en ce que** l'au moins un composé acide d) présente, à 20 °C, un solubilité dans du polyéther inférieure ou égale à 2 g/l, la notion de solubilité dans du polyéther désignant la solubilité, mesurée à 20 °C, du composé acide d) dans un polyether selon la formule suivante

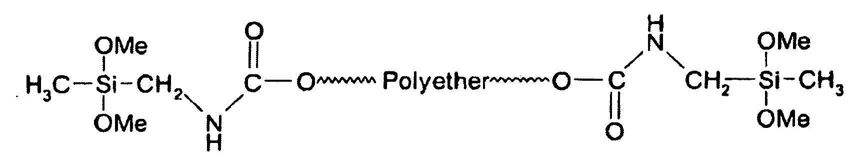

7. Matériau dentaire selon la revendication 6, **caractérisé en ce que** l'au moins un composé acide d) présente, à 20 °C, un solubilité dans du polyéther inférieure ou égale à 0,5 g/l, la notion de solubilité dans du polyéther désignant la solubilité, mesurée à 20 °C, du composé acide d) dans un polyéther selon la formule suivante

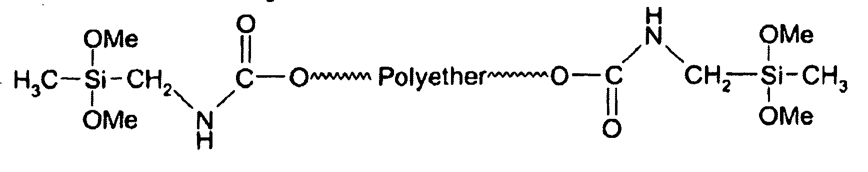

8. Matériau dentaire selon l'une des revendications précédentes, **caractérisé en ce que** l'au moins un composé acide d) présente, à 20 °C et mesuré dans l'eau, un pKₐ situé entre 1,0 et 6,5.

9. Matériau dentaire selon la revendication 8, **caractérisé en ce que** l'au moins un composé acide d) présente, à 20 °C et mesuré dans l'eau, un pKₐ situé entre 1,5 et 4,3.

10. Matériau dentaire selon l'une des revendications précédentes, **caractérisé en ce que** l'au moins un composé d) est choisi dans le groupe constitué d'acide barbiturique, d'anhydride succinique, d'acide éthylène-diamine-tétraacétique, de dérivés d'acide éthylène-diamine-tétraacétique, de sels mono-, di- et tri-alcalins d'acide éthylène-diamine-tétraacétique, de sels mono-, di-, tri-alcalinoterreux d'acide éthylène-diamine-tétraacétique et d'une quelconque de leurs combinaisons.

11. Matériau dentaire selon l'une des revendications précédentes, **caractérisé en ce que** ledit matériau dentaire contient, par rapport au mélange dans sa totalité, 0,001 à 10 % en poids de l'au moins un composé acide d).

12. Matériau dentaire selon la revendication 11, **caractérisé en ce que** ledit matériau dentaire contient, par rapport au mélange dans sa totalité, 0,2 à 1,5 % en poids de l'au moins un composé acide d).

13. Matériau dentaire selon l'une des revendications précédentes, **caractérisé en ce qu'**il contient, en tant que catalyseur b), une base organique dont le pK_{BH+}, mesuré dans de l'acétonitrile, est supérieur au égal à 20.

14. Matériau dentaire selon la revendication 13, **caractérisé en ce que** l'au moins une base catalytique b) est choisie dans le groupe constitué de 1,1,3,3-tétraméthylguanidine (TMG), de 1,8-diazabicyclo[5.4.0]undéc-7-ène (DBU), de 1,5-diazabicyclo[4.3.0]non-5-ène (DBN), de tert-butylimino-tris(diméthylamino)-phosphorane, de tert-butylimino-tri(pyrrolidino)phosphorane, de tert-octyliminotris(diméthylamino)phosphorane, de 2-tert-butylimino-2-diéthylamino-1,3-diméthylperhydro-1,3,2-diazaphosphorine, de 2-tert-butylimino-2-diéthylamino-1,3-diméthyl-perhydro-1,3,2-diazaphosphorine sur polystyrène, de 1-tert-butyl-2,2,4,4,4-pentakis(diéthylamino)-2Λ5,4Λ5-caténadi(phosphazène), de 1-éthyl-2,2,4,4,4-pentakis(diéthylamino)-2Λ5,4Λ5-caténadi(phosphazène), de 1-tert-butyl-4,4,4-tris(diméthylamino)-2,2-bis[tris-(diméthylamino)-phosphor-anylidenamino]-2Λ⁵,4Λ⁵-caténadi(phosphazène), de 1-tert-octyl-4,4,4-tris(diméthylamino)-2,2-bis[tris-(diméthylamino)-phosphor-anylidenamino]-2Λ⁵,4Λ⁵-caténadi(phosphazène), de 2,8,9-triisobutyl-2,5,8,9-tétraaza-1-phosphabicyclo[3.3.3]undécane, de 2,8,9-triisopropyl-2,5,8,9-tétraaza-1-phosphabicyclo[3.3.3]undécane, de 2,8,9-triméthyl-2,5,8,9-tétraaza-1-phosphabicyclo[3.3.3]undécane, de 2-tert-butyl-1,1,3,3-tétraméthylguanidine, de 1,8-bis(tétraméthylguanidino)naphtalène (TMGN), de 1,5,7-triazabicyclo(4.4.0)déc-5-ène, de 7-méthyl-1,5,7-triazabicyclo(4.4.0)déc-5-ène, de 1,5-diazabicyclo[4.3.0)non-5-ène et de 3,3,6,9,9-pentaméthyl-2,10-diazabicyclo-(4.4.0)déc-1-ène.

15. Matériau dentaire selon la revendication 13 ou 14, **caractérisé en ce qu'**il contient, par rapport au mélange dudit matériau dentaire dans sa totalité, 0,001 à 1 mmol/g d'une base catalytique b).

16. Matériau dentaire selon l'une des revendications 1 à 12, **caractérisé en ce qu'**il contient, en tant que catalyseur b), au moins un sel formé à partir d'au moins un cation choisi dans le groupe constitué de
- complexes de cations de métaux alcalins ou d'ammonium avec des éthers couronnes et/ou des cryptands,
- cations de tétraalkyl-, tétraaryl-, trialkylaryl-, dialkyldiaryl-, monoalkyltriarylammonium, cations de tétraalkyl-, tétraaryl-, trialkylaryl-, dialkyldiaryl-, monoalkyltriarylphosphonium, cations de tétraalkyl-, tétraaryl-, trialkylaryl-, dialkyldiaryl-, monoalkyltriarylarsonium, cations de tétraalkyl-, tétraaryl-, trialkylaryl-, dialkyldiaryl-, monoalkyltriarylstibonium,
- cations formés par protonation d'une base ayant un pK_{BH+}, mesuré dans de l'acétonitrile, d'au moins 20
et de leurs combinaisons, et d'au moins un anion d'un acide carboxylique (cyclo)aliphatique saturé et/ou insaturé, ledit acide carboxylique étant un acide carboxylique ramifié dont le groupe carboxyle est pourvu d'une chaîne (cyclo)alkyle d'une longueur d'au moins 2 atomes de carbone, ou un acide carboxylique linéaire dont le groupe carboxyle est pourvu d'une chaîne (cyclo)alkyle d'une longueur d'au moins 4 atomes de carbone.

17. Matériau dentaire selon la revendication 16, **caractérisé en ce que** ledit anion de l'au moins un sel catalytique b) est un anion d'un acide carboxylique ramifié dont le groupe carboxyle est pourvu d'une chaîne (cyclo)alkyle d'une longueur d'au moins 3 atomes de carbone ou d'un acide carboxylique linéaire dont le groupe carboxyle est pourvu d'une chaîne (cyclo)alkyle d'une longueur d'au moins 5 atomes de carbone.

18. Matériau dentaire selon la revendication 17, **caractérisé en ce que** ledit anion de l'au moins un sel catalytique b) est un anion d'acides monocarboxyliques cycloaliphatiques.

19. Matériau dentaire selon l'une des revendications 1 à 12, **caractérisé en ce qu'**il contient, en tant que catalyseur b), au moins un sel formé à partir d'une base organique faible dont le pK_{BH+}, mesuré dans l'eau, est situé entre -1 et 7, et d'au moins un acide fort dont le pKa, mesuré dans l'eau, est inférieur à 2.

20. Matériau dentaire selon la revendication 19, **caractérisé en ce que** l'au moins un catalyseur b) est un sel formé à partir d'une base organique faible dont le pK_{BH+}, mesuré dans l'eau, est situé entre -1 et 7, et d'au moins un acide fort dont le pKa, mesuré dans l'eau, est inférieur à 2, ledit acide présentant une structure permettant, suite à la déprotonation de l'acide, une stabilisation de la charge négative par mésomérie.

21. Matériau dentaire selon la revendication 19 ou 20, **caractérisé en ce que** l'on met en oeuvre au moins un sel catalytique b) qui est formé à partir d'au moins une base dont le pK_{BH+}, mesuré dans l'eau, est situé entre 1 et 7 et/ou d'au moins un acide dont le pKa, mesuré dans l'eau, est inférieur à 1.

22. Matériau dentaire selon la revendication 21, **caractérisé en ce que** l'on met en oeuvre au moins un sel catalytique b) qui est formé à partir d'au moins une base dont le pK_{BH+}, mesuré dans l'eau, est situé entre 3 et 6 et/ou d'au moins un acide dont le pKa, mesuré dans l'eau, est inférieur ou égal à 0,7.

23. Matériau dentaire selon l'une des revendications 19 à 22, **caractérisé en ce que** l'au moins un catalyseur b) est un sel formé à partir d'un acide choisi dans le groupe constitué d'acide p-toluène-sulfonique, d'acide fluorosulfonique, d'acide trifluorométhane-sulfonique, d'acide fluorosulfurique, d'acide 4-sulfophtalique, d'acide trichloroacétique, d'acide trifluoroacétique, d'acide benzène-sulfonique et de leurs combinaisons et/ou d'une base choisie dans le groupe constitué de dérivés de pyrrole, de diméthylaniline, de pyridine, de 2,4,6-N,N-pentaméthylaniline, de N,N-diméthylaniline, de phénétidine, d'acridine, de phénanthridine, de quinoléine, d'isoquinoléine, de 2-amino-4,6-diméthylpyrazine, de 4,6-diméthylpyridinamine, de 3-méthylpyridine (3-picoline), de 4-phénylpyridine, de 4-vinylpyridine, de pyridazine, de 2-éthylpyridine, de 2-butylpyridine, de 1,7-phénanthroline, de 2-aminopyrimidine, de 2-isopropylpyridine, de 2-vinylpyridine, de 2-N,N-diméthyl-aminopyridine, de quinazoline, de 4-chloropyridine, de phénazine, de 4-acétylpyridine, de méthylnicotinate, de 3-benzoylpyridine, de 2,2'-bipyridine, de 2-phénylpyridine, de 2-tert-butylpyridine, de pyrimidine, de 3-iodopyridine, de 3-fluoropyridine, de 3-chloropyridine, 3-bromopyridine, de pyrazine, de 7,8-benzoquinoléine, de 2-chloropyridine, de 4-cyanopyridine et de leurs combinaisons.

24. Mélange pouvant être obtenu en mélangeant les composants A et B dudit matériau dentaire à deux composants selon l'une des revendications 1 à 23, **caractérisé en ce que** le composant de base A est mélangé avec le composant catalytique B dans un rapport compris entre 1 : 1 et 20 : 1.

25. Utilisation d'un matériau dentaire selon l'une des revendications 1 à 23 pour la préparation de matériaux d'empreinte dentaires.
